(19) Europäisches Patentamt
European Patent Office
Office européen des brevets

(11) EP 1 115 427 B1

(12) **EUROPÄISCHE PATENTSCHRIFT**

(45) Veröffentlichungstag und Bekanntmachung des Hinweises auf die Patenterteilung:
**03.12.2003 Patentblatt 2003/49**

(21) Anmeldenummer: **99950545.6**

(22) Anmeldetag: **22.09.1999**

(51) Int Cl.[7]: **A61K 39/395**, A61K 35/14
// (C07K16/42, 16:28, 16:30)

(86) Internationale Anmeldenummer:
**PCT/EP99/07094**

(87) Internationale Veröffentlichungsnummer:
**WO 00/018435 (06.04.2000 Gazette 2000/14)**

(54) **VERWENDUNG VON TUMORZELLEN ZEITVERSETZT IN KOMBINATION MIT INTAKTEN ANTIKÖRPERN ZUR IMMUNISIERUNG**

TIME-STAGGERED UTILIZATION OF TUMOR CELLS IN COMBINATION WITH INTACT ANTIBODIES FOR IMMUNIZATION

UTILISATION DE CELLULES TUMORALES AVEC UN DECALAGE TEMPOREL EN COMBINAISON AVEC DES ANTICORPS INTACTS POUR L'IMMUNISATION

(84) Benannte Vertragsstaaten:
**AT BE CH CY DE DK ES FI FR GB GR IE IT LI LU MC NL PT SE**

(30) Priorität: **25.09.1998 DE 19844157**
**21.12.1998 DE 19859115**

(43) Veröffentlichungstag der Anmeldung:
**18.07.2001 Patentblatt 2001/29**

(73) Patentinhaber: **Lindhofer, Horst**
**82194 Gröbenzell (DE)**

(72) Erfinder:
- **LINDHOFER, Horst**
**D-82194 Gröbenzell (DE)**
- **RUF, Peter**
**D-82296 Schöngeising (DE)**

(74) Vertreter: **Reinhard - Skuhra - Weise & Partner**
**Friedrichstrasse 31**
**80801 München (DE)**

(56) Entgegenhaltungen:
**EP-A- 0 885 614** **DE-A- 19 710 497**

- **LINDHOFER H ET AL: "BISPECIFIC ANTIBODIES TARGET OPERATIONALLY TUMOR-SPECIFIC ANTIGENS IN TWO LEUKEMIA RELAPSE MODELS" BLOOD,US,PHILADELPHIA, PA, Bd. 88, Nr. 12, 15. Dezember 1996 (1996-12-15), Seiten 4651-4658, XP000616201 ISSN: 0006-4971**
- **CAMPBELL F A ET AL: "The role of tumor rejection antigens in host antitumor defense mechanisms." CANCER, (1995 JUN 1) 75 (11) 2649-55. REF: 88 , XP000877103**
- **LINDHOFER H ET AL: "BISPECIFIC ANTIBODIES EFFECTIVELY PURGE CANCER CELLS FROM PERIPHERAL BLOOD STEM CELL COLLECTIONS WITHOUT AFFECTING COLONY FORMING UNITS" ANNUAL MEETING INTERNATIONAL SOCIETY FOR EXPERIMENTAL HEMATOLOGY,XX,XX, Bd. 25, Nr. 8, 24. August 1997 (1997-08-24), Seite 879 XP002048523**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft die Verwendung von Tumorzellen zeitversetzt in Kombination mit intakten, bevorzugt heterologen Antikörpern zur Immunisierung von Mensch und Tier.

Die Immuntherapie durch Antikörper, insbesondere durch bispezifische oder trispezifische Antikörper, hat in den letzten Jahren an Bedeutung stetig zugenommen. Ein wesentliches Problem bei der Verwendung derartiger Antikörper in der Immuntherapie ist beispielsweise die Aktivierung der Immunzellen, z.B. der T-Lymphozyten.

In der DE 196 49 223 und der DE 197 10 495 sind intakte bispezifische und trispezifische Antikörper beschrieben, die zur Immuntherapie eingesetzt werden. Diese bispezfischen und trispezifischen Antikörper sind befähigt, an eine T-Zelle, zumindest ein Antigen auf einer Zielzelle und durch ihren Fc-Teil oder durch eine dritte Spezfität an Fc-Rezeptor positive Zellen (akzessorische Zellen) zu binden.

Es ist eine Aufgabe der vorliegenden Erfindung, ein neues Kombinationspräparat bereitzustellen, das sowohl Tumorzellen enthält, die so behandelt wurden, daß ihr Überleben nach Reinfusion verhindert wird, als auch intakte bispezifische und/trispezifische Antikörper, die gegen die Tumorzellen gerichtet sind.

Diese Aufgabe wird erfindungsgemäß dadurch gelöst, daß autologe Tumorzellen oder allogene Tumorzellen desselben Tumortyps, die je so behandelt wurden, daß ihr Überleben nach Reinfusion verhindert wird, zeitversetzt in Kombination mit intakten bispezifischen und/oder trispezifischen Antikörpern, die die nachfolgenden Eigenschaften aufweisen:

α) Binden an eine T-Zelle;
β) Binden an zumindest ein Antigen auf einer Tumorzelle;
γ) Binden durch ihren Fc-Teil (bei bispezifischen Antikörpern) oder durch eine dritte Spezifität (bei trispezifischen Antikörpern) an Fc-Rezeptor positiven Zellen,

an den zu immunisierenden Menschen verabreicht werden.

Die Tumorzellen und die Antikörper bilden erfindungsgemäß eine funktionelle Einheit und werden in Form einer Kombination verabreicht, um zielgerichtet eine Immunisierung zu erreichen, wobei es für den Erfolg der Verwendung darauf ankommt, daß ihre Anwendung zeitlich abgestuft voneinander erfolgt. Dabei ist es grundsätzlich möglich, zunächst die behandelten Tumorzellen zu verabreichen und, zeitlich abgesetzt hiervon, die Antikörper, oder zunächst die Antikörper an den Patienten zu verabreichen und, zeitlich abgestuft hiervon, die Tumorzellen.

Der Zeitraum, der zwischen der Verabreichung der Tumorzellen und der Antikörper und vice versa liegt, beträgt bevorzugt ca. 1 - 48 Stunden. Besonders bevorzugt ist ein Zeitraum von 1 - 24 Stunden, weiterhin bevorzugt 1 - 12 Stunden. Weiterhin möglich sind Zeiträume von 1 - 6 Stunden oder 2 - 4 Stunden. Entscheidend für den Erfolg der vorliegenden Erfindung ist die zeitlich abgestufte Anwendung, wobei der anzuwendende Zeitraum auch von der Art des zu behandelnden Tumors und vom verwendeten Antikörper abhängen kann. Die jeweiligen optimalen Zeiträume können anhand von Versuchen vom Fachmann ermittelt werden.

Die verwendeten Tumorzellen sollen möglichst intakt verabreicht werden. Es ist jedoch auch notwendig, daß ihr Überleben nach Reinfusion verhindert wird. Hierzu hat es sich als sinnvoll herausgestellt, die Tumorzellen entweder zu bestrahlen oder durch chemische Agentien am Überleben nach Reinfusion zu verhindern. Durch diese beiden Behandlungsarten bleibt insbesondere die äußere Struktur der Tumorzellen unverändert, d.h. das Erkennungsmuster für die Antikörper.

Zur Bestrahlung wird bevorzugt eine γ-Bestrahlung eingesetzt, die vorzugsweise mit einer Dosis von 20 - 200 Gy erfolgt. Bei einer chemischen Behandlung hat sich Mitomycin C besonders bewährt. Besonders bevorzugt werden heterologe bispezifische und/oder trispezifische Antikörper eingesetzt.

Der Immunisierungserfolg kann dadurch weiter verbessert werden, daß die Antikörper und die Tumorzellen, je zeitlich getrennt voneinander, mehrfach verabreicht werden. Eine weitere Verbesserung der Immunogenität der Tumorzellen ist dadurch erreichbar, daß diese vor Infusion einer Hitzevorbehandlung unterzogen werden. Der bevorzugte Bereich liegt hier bei 41 - 42°C, wobei das Optimum durch Versuche ermittelt werden kann. Bevorzugte Ergebnisse werden bei einer Temperatur von ca. 41,8°C erzielt. Der Zeitraum für die Hitzevorbehandlung beträgt im allgemeinen 1 bis 6 Stunden, bevorzugt ca. 3 Stunden. Der Zeitraum als auch die Temperatur, die optimalerweise eingesetzt werden können, hängen von der Art des zu behandelnden Tumors ab. Die jeweiligen Optima können vom Fachmann anhand von Laborversuchen ermittelt werden.

Wie im syngenen (autologen) murinen Tumormodell gezeigt werden konnte, spielen für eine besonders erfolgreiche Induktion der Immunität auch noch weitere Faktoren eine nicht unerhebliche Rolle. So ist die räumlich korrekte Präsentation des Tumormaterials wichtig. Die Tumorzellen müssen den für eine Immunisierung verantwortlichen Immunzellen im richtigen räumlichen Kontext präsentiert werden. Eine intravenöse (i.v.), intraperitoneale (i.p.) oder subkutane (s.c.) Applikation hat sich hierbei als besonders günstig herausgestellt, da hierdurch der optimale Kontakt in diesen Kompartimenten mit den entsprechenden Immunzellen unter Anwendung der bispezifischen und/oder trispezifischen Antikörper gewährleistet wird.

Zur erfolgreichen Immunisierung ist es weiterhin vorteilhaft, daß die Menge der verabreichten Tumorzellen in geeigneter Weise ausgewählt wird. So konnte in den Versuchen im murinen Tumormodell gezeigt werden, daß eine zu geringe Anzahl an Tumorzellen nicht den gewünschten Immunisierungserfolg bringt. Eine zu große Menge an Tumormaterial wiederum kann sich nachteilig auswirken, da beispielsweise Toleranzphänomene auftreten können. Überträgt man diese Resultate auf die Situation im Patienten, heißt dies, daß es für den Immunisierungserfolg vorteilhaft ist, eine definierte Menge an Tumormaterial im korrekten räumlichen Kontext mit einer ebenfalls definierten Menge an Antikörpern zu verabreichen. Ein Immunisierungserfolg stellt sich zwar auch dann ein, wenn einer dieser Parameter nicht optimiert wurde; besonders gute Ergebnisse werden jedoch erzielt, wenn sowohl die Mengen des Antikörpers als auch die Menge an Tumormaterial als auch der räumliche Kontext aufeinander abgestimmt und optimiert wurden.

Unter Berücksichtigung der obigen Ausführungen kann bei einer zu geringen Tumorzellzahl beispielsweise nur ein unzureichender Immunschutz etabliert werden. Daher ist es für einen vollständigen Immunisierungserfolg notwendig, mit einer definierten Anzahl an aktivierten Tumorzellen und einer definierten Menge an bispezifischen und/oder trispezifischen Antikörpern zu immunisieren. Die jeweiligen Zahlen können vom Fachmann anhand von Versuchen etabliert werden.

Die erfindungsgemäß eingesetzten bispezifischen und/oder trispezifischen Antikörper werden bevorzugt in einer Menge von 5 - 500 µg, weiterhin bevorzugt 10 - 300 µg, 10 - 100 µg oder 10 - 50 µg, je pro Infusion, verabreicht. Die Menge des eingesetzten Antikörpers hängt von der Art des zu behandelnden Tumors, von der Reaktion des Patienten und weiteren Faktoren ab. Die optimalen Mengen können vom Fachmann anhand von Versuchen ermittelt werden.

Die Tumorzellen werden bevorzugt in einer Menge von $10^7$ - $10^9$ Zellen pro Infusion verabreicht, wobei sich eine Zellzahl von ca. $10^8$ als bevorzugt herausgestellt hat. Die Tumorzellen wurden, wie oben ausgeführt, vor der Reinfusion so behandelt, daß ihr Überleben nach Reinfusion verhindert wird, wobei sie wahlweise zusätzlich einer Hitzevorbehandlung unterzogen werden können. Nähere Ausführungen hierzu sind in der vorliegenden Beschreibung enthalten.

Unumgänglich ist jedoch die zeitlich versetzte Verabreichung der Tumorzellen und der Antikörper. Dadurch wird gewährleistet, daß durch die Injektion des bispezifischen und/oder trispezifischen , bevorzugt heterologen Antikörpers in den zu behandelnden Tieren oder Menschen zunächst aufgrund des Überschusses an vorhandenen T-Lymphozyten im peripheren Blut diese über den hochaffinen Bindungsarm gebunden und präaktiviert werden. Zusätzlich können auch vorhandene Fc-Rezeptor positive akzessorische Immunzellen über den Fc-Anteil des bispezifischen oder trispezifischen Antikörpers gebunden werden. Wird nun dieser Zellkomplex aus T-Zelle und Fc-Rezeptor positiver Zelle über den zweiten hochaffinen Tumorbindungsarm an die vergleichsweise in geringer Anzahl vorhandenen Tumorzellen herangeführt, werden diese durch T-Lymphozyten bzw. Fc-Rezeptor positiven Zellen zerstört und über die in den Zellkomplex eingebundenen Fc-Rezeptor positiven Zellen, beispielsweise Makrophagen, phagozytiert. Dies konnte in einem Versuch mit markierten humanen Monozyten/Makrophagen und Tumorzellen bereits gezeigt werden. In einem nächsten Schritt wird das aufgenommene Tumormaterial prozessiert und über MHC Klasse I und insbesondere Klasse II Moleküle dem Immunsystem präsentiert, was eine wichtige Voraussetzung für die beobachtete humorale Immunantwort darstellt.

Wie das erfindungsgemäße Beispiel zeigt, setzt der Nachweis von tumorreaktiven Antikörpern, die nach einer Therapie gebildet wurden, die Aktivierung von CD4+ tumorspezifischen T-Lymphozyten voraus, die über die T-B-Zell-Kooperation tumorspezifische B-Zellklone stimulieren können. Durch den Nachweis der humoralen Immunantwort konnte somit indirekt ebenfalls eine zelluläre CD4-T-Zellantwort nachgewiesen werden und somit das Überleben der Mäuse als Folge der erfindungsgemäßen zeitlich versetzten Gabe von Tumorzellen und Antikörpern erklärt werden.

## BEISPIEL

In einem murinen, autologen Tumormodell wurde der Aufbau eines langanhaltenden Immunschutzes gegen den Tumor mit Hilfe von bsAk untersucht. Hierzu wurde C57BL/6 Mäusen zunächst eine an sich letale Dosis an autologen Melanomzellen verabreicht und zwei Tage später parentale bzw. bispezfische Antikörper nachinjiziert. Um zu testen, ob in den Mäusen hierbei ein langanhaltender Immunschutz aufgebaut worden ist, wurden die überlebenden Mäuse nach 100 Tagen einer erneuten Tumorgabe, diesmal jedoch ohne Ak, unterzogen. Um ferner der Frage nachzugehen, inwieweit für die Entwicklung des Immunschutzes die Anzahl an verabreichten Tumorzellen eine Rolle spielt, wurden die Versuche mit einer niedrigen Tumordosis von 1500 Tumorzellen (TZ) sowie mit einer hohen Dosis von 5000 Tumorzellen durchgeführt. Neben dem Überleben der Mäuse wurde darüber hinaus eine vorhandene humorale Immunantwort gegen den Tumor als Kriterium für einen vorliegenden Immunschutz herangezogen. Hierzu wurden die Seren der Mäuse vor und 100 Tage nach der Behandlung (unmittelbar vor der erneuten Tumorgabe) auf das Vorhandensein von anti-Tumorantikörpern miteinander verglichen.

Die Abbildung 1 zeigt die Überlebenskurven der Mäuse nach der primären Tumorgabe von 1500 bzw. 5000 Tumorzellen. Während die Kontrollmäuse ohne Antikörper-Behandlung alle innerhalb von 35 Tagen verstarben, konnten sämtliche mit bsAk behandelten Mäuse geheilt werden. Im Vergleich hierzu überlebten von den Mäusen, die mit einer Kombination aus den beiden parentalen Ak behandelt wurden, nur 84% nach der niedrigen Tumordosis und nur 16% nach der hohen Tumordosis.

Dieser qualitative Unterschied zwischen den bsAk und den parentalen Ak machte sich auch bei der Ausbildung einer humoralen Immunantwort bemerkbar. Die Abbildung 2 zeigt die in der Durchflußzytometrie gemessene humorale Immunantwort gegen die Tumorzellen. Während die Überlebenden aus der "parentalen Gruppe" keine humorale Immunantwort aufwiesen, zeigte sich dies bei den Mäusen aus der "bispezifischen Gruppe". Besonders wichtig hierfür war allerdings die Menge an verabreichtem Tumormaterial. Es gab einen signifikanten Unterschied zwischen den Gruppen mit niedrig- und hochdosierter Tumorgabe. Während nach niedriger verabreichter Tumordosis nur 17% der Mäuse einen starken und 33% einen schwachen Titer aufwiesen, konnte nach der höheren Tumordosis bei 66% der Mäuse ein starker Titer nachgewiesen werden.

Des weiteren korrelieren die gewonnenen Daten zur humoralen Immunantwort eindeutig mit dem Überleben der Mäuse nach erneuter Tumorgabe ohne nachfolgende Antikörper-Injektion. Nur die Mäuse, die einen starken Antikörper-Titer gegen den Tumor aufgebaut hatten, überlebten die Tumorgabe oder starben stark verzögert im Vergleich zur Kontrollgruppe ohne Immunisierung. Hingegen starben alle Mäuse ohne nachweisbaren Titer zuerst und ohne Verzögerung im Vergleich zur Kontrolle. In Abbildung 3 sind die Überlebenskurven der Mäuse dargestellt, die einer erneuten Tumorgabe unterzogen wurden ohne diese Mäuse anschließend wieder mit Antikörper zu behandeln. Die Mäuse, die eine hohe Primärgabe von 5000 TZ erhalten hatten, besaßen einen deutlichen Überlebensvorteil gegenüber den Mäusen mit einer niedrig dosierten Primärtumorgabe von 1500 TZ.

Nachfolgend werden bevorzugte Ausgestaltungen der Erfindung, insbesondere in Bezug auf die zu verwendenden bispezifischen und trispezifischen Antikörper und die Tumorzellen, offenbart.

Die Tumorzellen werden aus einem Patienten entnommen (autologe Tumorzellen). Um ein Überleben der Tumorzellen nach Reinfusion zu verhindern, werden sie vor dem Inkontaktbringen mit den Antikörpern, was ja erfindungsgemäß erst im Körper erfolgt, in an sich bekannter Weise behandelt, beispielsweise durch Bestrahlung. Erfindungsgemäß sind nicht beliebige Antikörper verwendbar, sondern diese müssen intakt sein, d.h. sie müssen eine funktionellen Fc-Teil besitzen. Bevorzugt sind sie heterologer Natur, d.h. die Antikörper sind aus schweren Immunglobulinketten unterschiedlicher Subklassen (-Kombinationen, auch Fragmente) und/oder Herkunft (Spezies) zusammengesetzt.

Diese intakten heterologen bispezifischen und/oder trispezifischen Antikörper werden so ausgewählt, daß sie weiterhin die nachfolgenden Eigenschaften aufweisen:

- α - Binden an eine T-Zelle;
- β - Binden an zumindest ein Antigen auf einer Tumorzelle;
- γ - Binden durch ihren Fc-Teil (bei bispezifischen Antikörpern) oder durch eine dritte Spezifität (bei trispezifischen Antikörpern) an Fc-Rezeptor positive Zellen.

Die erfindungsgemäß verwendbaren Antikörper sind befähigt, die Fc-Rezeptor positive Zelle zu aktivieren, wodurch die Expression von Zytokinen und/oder kostimulatorischen Antigenen initiiert oder erhöht wird.

Bei den trispezifischen Antikörpern erfolgt die Bindung an die Fc-Rezeptor positiven Zellen bevorzugt beispielsweise über den Fc-Rezeptor von Fc-Rezeptor positiven Zellen oder auch über andere Antigene auf Fc-Rezeptor positiven Zellen (Antigen-präsentierenden Zellen), wie z.B. den Mannose-Rezeptor.

Durch die erfindungsgemäße, zeitlich abgestufte Anwendung der intakten, bevorzugt heterologen bispezifischen und/oder trispezifischen Antikörper wird im Patienten eine Tumorimmunität aufgebaut, bevorzugt eine Langzeit-Tumorimmunität. Die Verabreichung (Reinfusion) erfolgt bevorzugt in einem Patienten nach der Behandlung des Primärtumors, bevorzugt bei Patienten in einer minimal residual disease (MRD)-Situation. Bei Patienten mit wenig verbliebenen Tumorzellen, bei denen allerdings die Gefahr eines Rezidivs hoch sein kann, ist die erfindungsgemäß beschriebene zeitlich abgestufte Anwendung besonders erfolgreich.

Die erfindungsgemaß verwendbaren heterologen bispezifischen und/oder trispezifischen Antikörper sind zum Teil an sich bekannt, zum Teil werden sie aber auch in der vorstehenden Anmeldung zum ersten Mal beschrieben. Ein Beispiel für einen bsAk ist der Antikörper anti-CD3 X anti-EpCAM (GA-733-2) der bei epithelialen Tumoren wie dem Mammacarcinom eingesetzt wird.

Auf der Tumorzelle erfolgt eine Hochregulation von MHC 1, sowie eine Aktivierung der intrazellulären Prozessierungsmaschinerie (Proteasom-Komplex) aufgrund der Freisetzung von Zytokinen (wie z.B. INF-γ und TNF-α) in unmittelbarer Nachbarschaft der Tumorzelle. Die Zytokine werden aufgrund bispezifischer Antikörpervermittelter Aktivierung von T-Zellen und akzessorischen Zellen freigesetzt. D.h. durch den intakten bsAk werden nicht nur Tumorzellen zerstört oder phagozytiert, sondern indirekt auch deren Tumorimmunogenität erhöht.

Die Aktivierung der Fc-Rezeptor positiven Zelle durch den bsAk ist von der Subklasse bzw. der Subklassenkombination des bsAk abhängig. Wie in in vitro-Versuchen nachgewiesen werden konnte, sind beispielsweise bsAk der Subklassenkombination Maus-IgG2a/-Ratte-IgG2b in der Lage, an Fc-Rezeptor positive Zellen zu binden und diese gleichzeitig zu aktivieren, was zur Hochregulation bzw. Neuausbildung (Expression) von kostimulatorischen Antigenen, wie z.B. CD40, CD80 oder CD86, auf der Zelloberfläche dieser Zellen führt. Dagegen sind bsAk der Subklassenkombination Maus-IgGl/IgG2b zwar in der Lage an Fc-Rezeptor positive Zellen zu binden (1) Haagen et al., J. Immunology, 1995, 154: 1852-1860, können diese aber offenbar nicht in vergleichbarem Maße aktivieren (2) Gast et al., Cancer Immunol. Immuntherap., 1995, 40: 390.

Während der intakte bsAk die T-Zelle mit einem Bindungsarm (z.B. an CD3 oder CD2) bindet und gleichzeitig aktiviert, können kostimulatorische Signale von der an den Fc-Teil des bsAk gebundenen Fc-Rezeptor positiven Zelle an die T-Zelle übermittelt werden. D.h. erst die Kombination von Aktivierung der T-Zelle über einen Bindungsarm des bsAk und der gleichzeitigen Übertragung von kostimulatorischen Signalen von der Fc-Rezeptor positiven Zelle auf die T-Zelle, führt zu einer effizienten T-Zellaktivierung (Abb. 4A). Tumor-spezifische T-Zellen, die an der Tumorzelle ungenügend aktiviert wurden und anergisch sind, können nach der erfindungsgemäßen ex vivo-Vorbehandlung ebenfalls reaktiviert werden (Abb. 4B).

Ein weiterer wichtiger Aspekt bei der Induktion einer Tumorimmunität ist die mögliche Phagozytose, Prozessierung und Präsentation von Tumorbestandteilen durch die vom bsAk herangeführten und aktivierten akzessorischen Zellen (Monozyten/Makrophagen, oder dendritische Zellen). Durch diesen klassischen Mechanismus der Präsentation von Antigenen können sowohl tumorspezifische CD4- wie auch CD8-positive Zellen generiert werden. Tumorspezifische CD4-Zellen spielen darüberhinaus eine wichtige Rolle für die Induktion einer humoralen Immunantwort im Zusammenhang mit der T-B-Zell Kooperation.

Bispezifische und trispezifische Antikörper können mit einem Bindungsarm an den T-Zellrezeptor-Komplex der T-Zelle, mit dem zweiten Bindungsarm an tumorassoziierte Antigene auf der Tumorzelle binden. Sie aktivieren dabei T-Zellen, die durch Freisetzung von Zytokinen oder Apoptose-vermittelnde Mechanismen die Tumorzellen zerstören. Darüber hinaus besteht offenbar die Möglichkeit, daß T-Zellen im Rahmen der Aktivierung mit bispezifischen Antikörpern tumorspezifische Antigene über ihren Rezeptor erkennen und dadurch eine dauerhafte Immunisierung eingeleitet wird (Abb. 4B). Von besonderer Bedeutung ist dabei der intakte Fc-Teil des bispezifischen oder trispezifischen Antikörpers, der die Bindung an akzessorische Zellen wie z.B. Monozyten/Makrophagen und dendritische Zellen vermittelt und diese veranlaßt selbst zytotoxisch zu werden und/oder gleichzeitig wichtige kostimulatorische Signale an die T-Zelle weiterzugeben. Auf diese Weise kann offensichtlich eine T-Zellantwort u.U. auch gegen bislang unbekannte, tumorspezifische Peptide induziert werden.

Durch Redirektion von u.U. anergisierten, tumorspezifischen T-Zellen an Tumorzellen mittels bispezifischer und/oder trispezifischer Antikörper bei gleichzeitiger Kostimulation derartiger T-Zellen durch akzessorische Zellen welche an den Fc-Teil des bispezifischen oder trispezifische Antikörpers binden, könnte die Anergie von zytotoxischen T-Zellen (CTLs) aufgehoben werden. D.h. eine im Patienten gegen den Tumor existierende T-Zell-Toleranz kann mittels intakter heterologer bispezifischer und/oder trispezifischer Antikörper gebrochen und damit eine dauerhafte Tumorimmunität induziert werden.

Für den letzten Punkt gibt es erste in vivo-Daten aus Mausversuchen, die auf eine derartige dauerhafte Tumorimmuntät nach Behandlung mit einem syngenen Tumor und intakten bsAk hinweisen. In diesen Versuchen überlebten insgesamt 14 von 14 Tieren, die nach einer ersten Tumorinjektion erfolgreich mit bsAk behandelt werden konnten, eine weitere Tumorinjektion 144 Tage nach der ersten Tumorinjektion - ohne eine erneute Gabe von bsAk.

Die erfindungsgemäß eingesetzten Antikörper sind bevorzugt zur Reaktivierung von in Anergie befindlichen, tumorspezifischen T-Zellen befähigt. Weiterhin sind sie zur Induktion von tumorreaktiven Komplement-bindenden Antikörpern und damit zur Induktion einer humoralen Immunantwort in der Lage.

Die Bindung erfolgt bevorzugt über CD3, CD2, CD4, CD5, CD6, CD8, CD28 und/oder CD44 an die T-Zelle. Die Fc-Rezeptor positiven Zellen weisen zumindest einen Fcγ-Rezeptor I, II oder III auf.

Erfindungsgemäß einsetzbare Antikörper sind zur Bindung an Monozyten, Makrophagen, dendritische Zellen, "Natural Killer"-Zellen (NK-Zellen) und/oder aktivierte neutrophile Zellen als Fcγ-Rezeptor 1 und/oder III positive Zellen befähigt.

Die erfindungsgemäß einsetzbaren Antikörper bewirken, daß die Expression von CD40, CD80, CD86, ICAM-1 und/oder LFA-3 als kostimulatorische Antigene oder/und die Sekretion von Zytokinen durch die Fc-Rezeptor positive Zelle initiiert oder erhöht wird. Die Zytokine sind bevorzugt IL-1, IL-2, IL-4, IL-6, IL-8,IL-12, INF-γ und/oder TNF-α.

Die Bindung an die T-Zelle erfolgt bevorzugt über den T-Zell-Rezeptor-Komplex der T-Zelle.

Die erfindungsgemäß einsetzbaren bispezifischen Antikörper sind beispielsweise:

- ein anti-CD3 X anti-Tumor-assoziiertes Antigen- und/oder anti-CD4 X anti-Tumor-assoziiertes Antigen- und/oder anti-CD5 X anti-Tumor-assoziiertes Antigen- und/oder anti-CD6 X anti-Tumor-assoziiertes Antigen- und/oder anti-CD8 X anti-Tumor-assoziiertes Antigen- und/oder anti-CD2 X anti-Tumor-assoziiertes Antigen- und/oder anti-CD28 X anti-Tumor-assoziiertes Antigen- und/oder anti-CD44 X anti-Tumor-assoziiertes Antigen-Antikörper ist.

Die erfindungsgemäß einsetzbaren trispezifischen Antikörper sind bevorzugt:

- ein anti-CD3 X anti-Tumor-assoziiertes Antigen- und/oder anti-CD4 X anti-Tumor-assoziiertes Antigen- und/oder anti-CD5 X anti-Tumor-assoziiertes Antigen- und/oder anti-CD6 X anti-Tumor-assoziiertes Antigen- und/oder anti-CD8 X anti-Tumor-assoziiertes Antigen- und/oder anti-CD2 X anti-Tumor-assoziiertes Antigen- und/oder anti-CD28 X anti-Tumor-assoziiertes Antigen- und/oder anti-CD44 X anti-Tumor-assoziiertes Antigen-Antikörper.

Die erfindungsgemäß einsetzbaren trispezfischen Antikörper weisen zumindest eine T-Zell-Bindungsarm, einen Tumorzell-Bindungsarm und einen an Fc-Rezeptor positive Zellen bindenden Bindungsarm auf. Dieser zuletzt genannte Bindungsarm kann ein anti-Fc-Rezeptor-Bindungsarm oder ein Mannose-Rezeptor-Bindungsarm sein.

Der bispezifische Antikörper ist bevorzugt ein heterologer intakter Ratte/Maus bispezifischer Antikörper.

Mit den erfindungsgemäß einsetzbaren bispezifischen und trispezifischen Antikörpern werden T-Zellen aktiviert und gegen die Tumorzellen redirigiert. Bevorzugt einsetzbare heterologe intakte bispezifische Antikörper werden aus einer oder mehreren der nachfolgenden Isotyp-Kombinationen ausgewählt:

Ratte-IgG2b/Maus-IgG2a,
Ratte-IgG2b/Maus-IgG2b,
Ratte-IgG2b/Maus-IgG3,

Ratte-IgG2b/Human-IgGl,
Ratte-IgG2b/Human-IgG2,
Ratte-IgG2b/Human-IgG3[orientaler Allotyp G3m(st) = Bindung an Protein A],
Ratte-IgG2b/Human-IgG4,

Ratte-IgG2b/Ratte-IgG2c,

Maus-IgG2a/Human-IgG3[kaukasische Allotypen G3m(b+g) = keine Bindung an Protein A, im folgenden mit * gekennzeichnet]

Maus-IgG2a/Maus-[VH-CH1,VL-CL]-Human-IgG1-[Hinge]-Human-IgG3*-[CH2-CH3]

Maus-IgG2a/Ratte-[VH-CH1,VL-CL]-Human-IgG1-[Hinge]-Human-IgG3*-[CH2-CH3]

Maus-IgG2a/Human-[VH-CH1,VL-CL]-Human-IgG1-[Hinge]-Human-IgG3*-[CH2-CH3]

Maus-[VH-CH1,VL-CL]-Human-IgG1/Ratte-[VH-CH1,VL-CL]-Human-IgG1-[Hinge]-Human-IgG3*-[CH2-CH3]

Maus-[VH-CH1,VL-CL]-Human-IgG4/Ratte-[VH-CH1,VL-CL]-Human-IgG4-[Hinge]-Human-IgG4[N-terminale Region von CH2]-Human-IgG3*[C-terminale Region von CH2: > AS-Position 251]-Human- IgG3*[CH3]

Ratte-IgG2b/Maus-[VH-CH1,VL-CL]-Human-IgG1-[Hinge-CH2-CH3]

Ratte-IgG2b/Maus-[VH-CH1,VL-CL]-Human-IgG2-[Hinge-CH2-CH3]

Ratte-IgG2b/Maus-[VH-CH1,VL-CL]-Human-IgG3-[Hinge-CH2-CH3, orientaler Allotyp]

Ratte-IgG2b/Maus-[VH-CH1,VL-CL]-Human-IgG4-[Hinge-CH2-CH3]

Human-IgG1/Human-[VH-CH1,VL-CL]-Human-IgG1-[Hinge]-Human-IgG3*-[CH2-CH3]

Human-IgG1/Ratte-[VH-CH1,VL-CL]-Human-IgG1-[Hinge]-Human-IgG4[N-terminale Region von CH2]-Human-IgG3*[C-terminale Region von CH2 : > AS-Position 251]-Human-IgG3*[CH3]

Human-IgG1/Maus-[VH-CH1,VL-CL]-Human-IgG1-[Hinge]-Human-IgG4[N-terminale Region von CH2]-Human-IgG3*[C-terminale Region von CH2 : > AS-Position 251]-Human-IgG3*[CH3]

Human-IgG1/Ratte-[VH-CH1,VL-CL]-Human-IgG1-[Hinge]-Human-IgG2[N-terminale Region von CH2]-Human-IgG3*[C-terminale Region von CH2 : > AS-Position 251]-Human-IgG3*[CH3]

Human-IgG1/Maus-[VH-CH1,VL-CL]-Human-IgG1-[Hinge]-Human-IgG2[N-terminale Region von CH2]-Human-IgG3*[C-terminale Region von CH2 : > AS-Position 251]-Human-IgG3*[CH3]

Human-IgG1/Ratte-[VH-CH1,VL-CL]-Human-IgG1-[Hinge]-Human-IgG3*-[CH2-CH3]

Human-IgG1/Maus-[VH-CH1,VL-CL]-Human-IgG1-[Hinge]-Human-IgG3*-[CH2-CH3]

Human-IgG2/Human-[VH-CH1,VL-CL]-Human-IgG2-[Hinge]-Human-IgG3*-[CH2-CH3]

Human-IgG4/Human-[VH-CH1,VL-CL]-Human-IgG4-[Hinge]-Human-IgG3*-[CH2-CH3]

Human-IgG4/Human-[VH-CH1,VL-CL]-Human-IgG4-[Hinge]-Human-IgG4[N-terminale Region von CH2]-Human-IgG3*[C-terminale Region von CH2 : > AS-Position 251]-Human-IgG3*[CH3]

Maus-IgG2b/Ratte-[VH-CH1,VL-CL]-Human-IgG1-[Hinge]-Human-IgG3*-[CH2-CH3]

Maus-IgG2b/Human-[VH-CH1,VL-CL]-Human-IgG1-[Hinge]-Human-IgG3*-[CH2-CH3]

Maus-IgG2b/Maus-[VH-CH1,VL-CL]-Human-IgG1-[Hinge]-Human-IgG3*-[CH2-CH3]

Maus-[VH-CH1,VL-CL]-Human-IgG4/Ratte-[VH-CH1,VL-CL]-Human-IgG4-[Hinge]-Human-IgG4-[CH2]-Human-IgG3*-[CH3]

HumanIgG1/Ratte[VH-CH1,VL-CL]-Human-IgG1[Hinge]-Human-IgG4-[CH2]-HumanIgG3*[CH3]

HumanIgG1/Maus[VH-CH1,VL-CL]-Human-IgG1[Hinge]-Human-IgG4-[CH2]-Human-IgG3*[CH3]

Human-IgG4/Human[VH-CH1,VL-CL]-Human-IgG4-[Hinge]-Human-IgG4-[CH2]-HumanIgG3*-[CH3]

Bei den erfindungsgemäß verwendbaren Antikörpern handelt es sich vorzugsweise um monoklonale, chimäre, rekombinante, synthetische, halbsynthetische oder chemisch modifizierte intakte Antikörper mit beispielsweise Fv-, Fab-, scFv- oder $F(ab)_2$-Fragmenten.

Bevorzugt werden Antikörper oder Derivate oder Fragmente vom Menschen verwendet oder solche, die derart verändert sind, daß sie sich für die Anwendung beim Menschen eignen (sogenannte "humanized antibodies") (siehe z.B. Shalaby et al., J. Exp. Med. 175 (1992), 217; Mocikat et al., Transplantation 57 (1994), 405).

Die Herstellung der verschiedenen oben genannten Antikörpertypen und -fragmente ist dem Fachmann geläufig. Die Herstellung monoklonaler Antikörper, die ihren Ursprung vorzugsweise in Säugetieren, z.B. Mensch, Ratte, Maus, Kaninchen oder Ziege, haben, kann mittels herkömmlicher Methoden erfolgen, wie sie z.B. in Köhler und Milstein (Nature 256 (1975), 495), in Harlow und Lane (Antibodies, A Laboratory Manual (1988), Cold Spring Harbor) oder in Galfré (Meth. Enzymol. 73 (1981), 3) oder der DE 195 31 346 beschrieben sind.

Ferner ist es möglich, die beschriebenen Antikörper mittels rekombinanter DNA-Technologie nach dem Fachmann geläufigen Techniken herzustellen (siehe Kurucz et al., J. Immunol. 154 (1995), 4576; Holliger et al., Proc. Natl. Acad. Sc. USA 90 (1993), 6444).

Die Herstellung von Antikörpern mit zwei verschiedenen Spezifitäten, den sogenannten bispezifischen Antikörpern, ist zum einen durch Anwendung rekombinanter DNA-Technologie möglich, aber auch durch die sogenannte Hybrid-Hybridoma-Fusionstechnik (siehe z.B. Milstein et al., Nature 305 (1983), 537). Hierbei werden Hybridomazellinien, die Antikörper mit jeweils einer der gewünschten Spezifitäten produzieren, fusioniert und rekombinante Zellinien identifiziert und isoliert, die Antikörper mit beiden Spezifitäten produzieren.

Das der Erfindung zugrunde liegende Problem kann sowohl durch bispezifische als auch trispezifische Antikörper gelöst werden, sofern sie die im Anspruch 1 gekennzeichneten Eigenschaften und Wirkungen aufweisen. Nachfolgend wird die Herstellung von Antikörpern mit Zwei- und Dreispezifitäten näher beschrieben. Die Bereitstellung derartiger bispezifischer und trispezifischer Antikörper gehört zum Stand der Technik, und auf die derartige Herstellungstechniken beschreibende Literatur wird hier voll inhaltlich Bezug genommen.

Die Herstellung von Antikörpern mit drei Spezifitäten, sogenannten trispezifischen Antikörpern, durch die das der Erfindung zugrundeliegende Problem ebenfalls lösbar ist, kann beispielsweise derart erfolgen, daß an eine der schweren Ig-Ketten eines bispezifischen Antikörpers eine dritte Antigenbindungsstelle mit einer weiteren Spezifität, z. B. in Form eines "single chain variable fragments" (scFv), angekoppelt wird. Das scFv kann beispielsweise über einen

$-S-S(G_4S)_nD-I-Linker$

an eine der schweren Ketten gebunden sein (S = Serin, G = Glycin, D = Aspartat, I = Isoleucin).

Analog dazu können trispezifische $F(ab)_2$-Konstrukte hergestellt werden, indem die CH2-CH3-Regionen der schweren Kette einer Spezifität eines bispezifischen Antikörpers ersetzt werden durch ein scFv mit einer dritten Spezifität, während die CH2-CH3-Regionen der schweren Kette der anderen Spezifität beispielsweise durch Einbau eines Stopcodons (am Ende der "Hinge"-Region) in das codierende Gen, z.B. mittels homologer Rekombination, entfernt werden (siehe Abb.5).

Möglich ist auch die Herstellung trispezifischer scFv-Konstrukte. Hierbei werden drei VH-VL-Regionen, die drei verschiedene Spezifitäten repräsentieren, hintereinander in Reihe angeordnet (Abb.6).

Erfindungsgemäß werden z.B. intakte bispezifische Antikörper verwendet. Intakte bispezifische Antikörper sind aus zwei Antikörper-Halbmolekülen (je eine H- und L-Immunglobulinkette), die jeweils eine Spezifität repräsentieren, zusammengesetzt, und besitzen darüber hinaus, wie normale Antikörper auch, einen Fc-Teil mit den bekannten Effektorfunktionen. Sie werden bevorzugt durch die Quadrom-Technologie hergestellt. Dieses Herstellungsverfahren ist beispielhaft in der DE-A-44 19 399 beschrieben. Auf diese Druckschrift, auch bzgl. einer Definition der bispezifischen Antikörper, wird zur vollständigen Offenbarung vollinhaltlich Bezug genommen. Selbstverständlich sind auch andere Herstellungsverfahren einsetzbar, solange sie zu den erfindungsgemäß notwendigen intakten bispezifischen Antikörpern der obigen Definition führen.

Beispielsweise können in einem neu entwickelten Herstellungsverfahren (6) intakte bispezifische Antikörper in ausreichender Menge produziert werden. Die Kombination von 2 bispezifischen Antikörpern gegen 2 unterschiedliche tumorassoziierte Antigene (z.B. c-erb-B2, ep-cam, beispielsweise GA-733-2 = C215) auf den Mammakarzinomzellen minimiert die Gefahr, daß Tumorzellen, die nur ein Antigen exprimieren, unerkannt bleiben.

Es wurde auch versucht, durch Behandlung mit bispezifischen F(ab')2-Fragmenten mit den Spezifitäten anti-c-erb-B2 x antiCD64 eine Tumorimmunität zu erreichen. Der Hauptnachteil von bsF(ab')2-Fragmenten liegt darin, daß aufgrund der verwendeten Spezifitäten lediglich FcγRI+ Zellen an den Tumor redirigiert werden. T-Zellen werden durch diesen bispezifischen Antikörper nicht an den Tumor redirigiert. Die FcγRI+ Zellen können zwar indirekt durch Präsentation von tumorspezifischen Peptiden (über MHC I bzw. MHCII) nach z.B. Phagozytose von Tumorzellbestandteilen tumorspezifische T-Zellen aktivieren, die Effizienz der Induktion einer Tumorimmunität ist hier aber niedriger , da T-Zellen durch diesen bsAK nicht gebunden werden und auch nicht zur Kostimulation der Fc-Rezeptor positiven Zellen beitragen können.

Weitere Vorteile von intakten bsAk mit der Fähigkeit zur Redirektion von T-Zellen gegenüber den o.g. bsF (ab')2 Fragmenten sind im einzelnen:

1. An intakte bsAk können Fc-Rezeptor positive Zellen binden und einerseits über ADCC (antibody-dependent cell-mediated cytotoxicity) direkt zur Tumorzerstörung beitragen sowie andererseits wie oben näher ausgeführt zur T-Zellaktivierung.

2. Durch intakte T-Zell-redirigierende bsAk werden auch anergisierte tumorspezifische T-Zellen an die Tumorzelle herangeführt, die erfindungsgemäß direkt am Tumor reaktiviert werden können. Dies kann durch ein bsF(ab') 2-Fragment mit den Spezifitäten anti-CD64Xanti-tumorassoziiertes Antigen nicht erreicht werden.

3. Ein bsF(ab')2-Fragment mit den Spezifitäten anti-CD64Xanti-tumorassoziiertes Antigen kann lediglich eine Tumorimmunität in 30% der Patienten erzielen, während erfindungsgemäß in Mausversuchen mit T-Zell-redirigierenden intakten bsAk ein Schutz in 100% der Tiere erzielt werden konnte.

Die Bindung des bsAk an Fcγ-RI besitzt zwei wesentliche Vorteile im Hinblick auf eine optimale anti-Tumorwirksamkeit:

(1) Fcγ-RI positive Zellen besitzen die Fähigkeit mittels ADCC Tumorzellen zu eliminieren und können insofern synergistisch zur anti-Tumorwirkung der durch den bsAk an die Tumorzelle herangeführten cytotoxischen T-Zellen beitragen.

(2) Fcγ-RI positive Zellen (wie z.B. Monozyten/Makrophagen/-Dendriten) sind in der Lage, wichtige kostimulatorische Signale, ähnlich wie bei der Antigen-Präsentation, der T-Zelle zu liefern und damit eine Anergisierung der T-Zelle zu verhindern. Wie in Abbildung 4 gezeigt können weiterhin, als erwünschtes Nebenprodukt, aufgrund der durch intakten bsAk vermittelten Interaktion von T-Zelle mit akzessorischer Zelle und Tumorzelle sogar T-Zellen, deren T-Zellrezeptor tumorspezifische Peptide (über MHC Antigene auf der Tumorzelle präsentiert) erkennt, stimuliert werden. Die für eine korrekte Aktivierung der T-Zelle notwendigen Kostimuli würden in dieser Konstellation von der akzessorischen Zelle (z.B. Monocyt) geliefert werden. Insofern sollte der erfindungsgemäße Antikörper neben der direkten T-Zellrezeptor-unabhängigen, durch bsAk vermittelten Tumorzerstörung (Abb.4A) ebenfalls tumorspezifische T-Zellen aktivieren und generieren (Abb.4B), die nach Abbau der bsAk weiterhin im Patienten patrouillieren. D.h. mittels intakter bsAk kann ähnlich wie bei gentherapeutischen Ansätzen (z.B. durch Einbau von kostimulatorischen Antigenen wie B-7 in die Tumorzelle) die Tumortoleranz im Patienten durchbrochen werden.

Günstig in diesem Zusammenhang ist weiterhin, daß die Expression von Fcγ-RI nach G-CSF -Behandlung auf den entsprechenden Zellen hochreguliert wird.

Die erfindungsgemäß eingesetzten intakten, bevorzugt herterologen spezifischen und/oder trispezifischen Antikörper weisen die nachfolgenden Eigenschaften auf:

a) Binden an eine T-Zelle;
b) Binden an zumindest ein Antigen auf einer Tumorzelle;
c) Binden durch ihren Fc-Teil (bei bispezifischen Antikörpern) oder durch eine dritte Spezifität (bei trispezifischen Antikörpern) an Fc-Rezeptor positive Zellen

und sie induzieren hierdurch eine Immunantwort und/oder zerstörte Tumorzellen, wobei der Antikörper so ausgewählt ist, daß er an ein Oberflächenantigen als Zielantigen auf der Tumorzelle bindet, welches induzierbar ist und im nicht induzierten Zustand (Normalzustand) auf der Zelle nicht vorkommt oder in einer derart geringen Zahl, daß die Anzahl für eine Zerstörung der Zelle nicht ausreicht. (Abb. 7A + B)

Es konnte gezeigt werden, daß nach Induktion bereits eine relativ geringe Anzahl der Zielantigene auf der Tumorzelle ausreichend ist, um die intakten bispezifischen und trispezifischen Antikörper zu binden und eine Zerstörung der Tumorzelle und/oder eine Immunantwort einzuleiten. Unter "geringe Anzahl" wird erfindungsgemäß eine Anzahl von Zielantigenen auf der Tumorzelle von mehr als 100 Zielantigenen pro Tumorzelle, bevorzugt mehr als 300 und weiterhin bevorzugt mehr als 1000 Zielantigenen pro Zelle verstanden. Die induzierbaren Zielantigene können auch in einer Menge von bis zu 500.000 pro Zielzelle vorliegen, wobei auch Mengen bis zu 400.000, bis zu 300.000, bis zu 200.000 oder bis zu 100.000 pro Zielzelle induzierbar sind. Ein weiterer bevorzugter Mengenbereich, in dem die Zielantigene vorliegen können, ist von 50.000 bis 100.000 und von 5000 bis 50.000.

Beispiele für induzierbare Oberflächenantigene auf Tumorzellen (Zielantigene), die für eine Immuntherapie durch intakte bispezifische und trispezifische Antikörper einsetzbar sind, sind Hitzeschock-Proteine und "MHC-Klasse I-verwandte" MIC-Moleküle. Hitzeschock-Proteine (Hsp) werden von der Zelle als Antwort auf Zellstreß synthetisiert. Unter "Zellstreß" ist erfindungsgemäß beispielsweise eine Entartung der Zelle, das Einwirken von Strahlen, chemischen Substanzen und von erhöhter Temperatur auf die Zelle sowie die Infektion der Zelle durch Mikroorganismen zu subsumieren. Zur Zeit sind vier Familien von Hitzeschock-Proteinen bekannt, die aufgrund ihres unterschiedlichen Molekulargewichts differenziert werden. Diese Familien werden als Hsp25, Hsp60, Hsp70 und Hsp90 bezeichnet, wobei die Zahl das ungefähre Molekulargewicht der Streßproteine in Kilo-Dalton wiedergibt. Die Hitzeschock-Proteine zeichnen sich durch hochkonservierte Aminosäuresequenzen aus, wobei der Grad der Konservierung bei über 35 % Aminosäure-Identität, bevorzugt bei 35 - 55 %, weiterhin bevorzugt 55 - 75% und am bevorzugtesten zwischen 75 % bis 85 % Aminosäure-Identität liegt.

Auf folgende Übersichtsartikel wird verwiesen: Annu. Rev. Genet. 27, 437-496 (1993); Biochimie 76, 737-747 (1994); Cell. Mol. Life Sci. 53, 80-129, 168-211 (1997); Experientia 48, 621-656 (1992); Kabakov u. Gabai, Heat Shock Proteins and Cytoprotection, Berlin: Springer 1997.

Hitzeschock-Proteine werden im Normalfall auf gesundem Gewebe nicht exprimiert. Es gibt jedoch Untersuchungen, die zeigen, daß Hitzeschock-Proteine beispielsweise auf Tumorzellinien und auf Tumormaterial aus Patienten exprimiert werden können ( Melcher et al., Nature Medicine, 4:581, 1998). Die Expression der Hitzeschock-Proteine auf den Tumorzellinien ist aber relativ schwach und deshalb für bisher bekannte immuntherapeutische Ansätze mit monoklonalen Antikörpern und unvollständigen (F(ab)2) bispezifischen Antikörpern ungeeignet.

Beispiele für Hitzeschock-Proteine sind Hsp60 und Hsp70/72.

Gleiches gilt für MIC-Moleküle. Auch diese Moleküle sind durch Zellstreß, wie oben näher definiert, induzierbar. MIC-Moleküle sind MHC I-verwandte Moleküle, die unter Kontrolle von Hitzeschock-Promoter-Elementen stehen (Groh et al., PNAS 93: 12445, 1996). Beispiele für MIC-Moleküle sind MIC A und MIC B. Auch für MIC-Moleküle konnte gezeigt werden, daß sie auf Normalgewebe nicht oder nur in so geringer Menge exprimiert werden, daß sie als Zielstruktur zur Erkennung durch einen Antikörper, um eine Zerstörung der Zellen zu erreichen, nicht geeignet sind, während sie auf beispielsweise epithelialen Tumoren in einer solchen Menge exprimiert werden, daß sie durch die erfindungsgemäß bereitgestellten bispezifischen und trispezifischen Antikörper bei einer Immuntherapie als Zielantigene einsetzbar sind.

Die erfindungsgemäß bereitgestellten intakten bispezifischen oder trispezifischen Antikörper werden so ausgewählt, daß sie gegen zumindest ein Zielantigen auf einer Tumorzelle gerichtet sind, welches induzierbar ist und auf der Tumorzelle im nicht induzierten Zustand nicht oder im wesentlichen nicht vorkommt. Diese Zahl liegt beispielsweise bei ca. 100 Zielantigenen/Zelle. Dies heißt jedoch nicht, daß derartige Zielantigene auf anderen Zellen, d.h. Nicht-Zielzellen, nicht vorkommen können. Beispielsweise kommen auf sich schnell regenerierendem Gewebe, z.B. den Schleimhautzellen des Magen-Darm-Traktes, auch im physiologischen Zustand, konstitutiv exprimiert, Hitzeschockproteine vor. Diese Hitzeschockproteine werden jedoch von der vorliegenden Erfindung nicht mit umfaßt, da sie nicht induzierbar sind, sondern bereits auf Normalgewebe vorkommen. Bei Verwendung der erfindungsgemäßen Antikörper, die gegen Hitzeschockproteine gerichtet sind, können auch bestimmte Schleimhautzellen des Magen-Darm-Traktes zerstört werden, da diese, wie oben ausgeführt, konstitutiv Hitzeschockproteine auf ihrer Zelloberfläche exprimieren. Die mögliche Zerstörung dieser Zellen ist für einen Patienten zwar mit gewissen Nachteilen verbunden, die jedoch im Vergleich zur erzielbaren Tumorzerstörung gering anzusetzen sind.

Das sich schnell regenerierende Gewebe ist somit nicht primäres Target der erfindungsgemäßen Antikörper, kann jedoch dann von den Antikörpern "getroffen" werden, wenn der erfindungsgemäße Antikörper nicht nur die induzierbaren Zielantigene auf der Tumorzelle erkennt, sondern diese Antigene z.B. auch auf sich schnell regenerierendem Gewebe vorkommen. Da sich dieses Gewebe jedoch schnell teilt, kann nach Absetzen der Antikörper-Therapie der ursprüngliche Zustand dieses Gewebes relativ rasch so wieder hergestellt werden, daß es seine physiologische Aufgabe wieder erfüllen kann. Die Erfindung ist also nicht darauf gerichtet, daß die erfindungsgemäßen bispezifischen und trispezifischen Antikörper Antigene auf z.B. sich schnell regenerierendem Gewebe erkennen, welche dort unter Umständen konstitutiv exprimiert werden, sondern die Erfindung umfaßt ausschließlich solche Antikörper, die gegen Zielantigene gerichtet sind, die auf der Tumorzelle induzierbar sind und nach Induktion, beispielsweise auch konstitutiv, exprimierbar sind.

Erfindungsgemäß werden unter "induzierbar" solche Zielantigene verstanden, die hier operationell tumorspezifisch sind und im physiologischen Zustand auf der Zelle nicht oder nur in einer solchen Anzahl vorkommen, daß eine Immunantwort gegen sie nicht induzierbar ist oder eine Zerstörung der Zellen aufgrund dieser geringen Anzahl nicht oder in einem nicht wesentlichen, therapeutisch nutzbaren Umfang stattfindet.

Die erfindungsgemäß bereitgestellten intakten bispezifischen und trispezifischen Antikörper führen nicht nur einen Typ von Immunzellen, sondern T-Zellen und akzessorische Zellen an die Tumorzelle heran, und sie sind aus diesem Grund für die Erkennung induzierbarer Oberflächenantigene als operationelle Zielstrukturen besonders geeignet. Es konnte gezeigt werden, daß bereits wenige Zielantigene in einer Menge von 100 bis 5000 pro Zelle auf der Tumorzelle ausreichend sind, um diese zu zerstören. Die hierzu durchgeführten in vitro-Experimente mit Stammzellpräparaten (PBSZ) sind im nachfolgenden Beispiel A beschrieben. Somit ist die erfindungsgemäße Klasse intakter bispezifischer und trispezifischer Antikörper befähigt, auch bei einer sehr geringen Expression der Zielantigene Tumorzellen zu zerstören oder nach deren Erkennung eine Immunantwort zu initiieren.

Die erfindungsgemäßen Antikörper können, da sie gegen induzierbare Antigene gerichtet sind, dazu beitragen, das Problem fehlender zielzellspezifischer, für eine Immuntherapie notwendige Zielantigene auf Tumorzellen zu lösen.

**BEISPIEL 1**

Nachdem in präklinischen Tiermodellen die herausrangende Wirksamkeit von intakten bsAk nicht nur in vitro, sondern auch in vivo belegt werden konnte (Lindhofer et al., Blood, 88:4651, 1996), wurde die Reinigung von Stammzellpräparaten von kontaminierenden Tumorzellen als weitere Anwendungsmöglichkeit entwikkelt (Lindhofer et al., Exp. Hematol. 25:879, 1997).

Auf diesen Versuchen aufbauend, konnte in Folgeversuchen mit kompletten Stammzellpräparaten (ca. 2 x 10e10 Zellen), insbesondere die Wirksamkeit im untergesättigten Bereich der Zielantigene nachgewiesen werden. D. h. es wurde, in Titrationsversuchen, so wenig intakter bsAk verabreicht, daß in einem PBSZ (Periphere Blut-Stammzellen)-Präparat mit ca. 6,5 x 10e9 T-Zellen, bei einer Gabe von 5µg Antiköper/Präparat, lediglich 3000 CD3 Moleküle von ca. 30.000 CD3 Molekülen/T-Zelle mit dem bsAk gebunden vorlagen (siehe Berechnung). Trotzdem waren die intakten bsAk in der Lage, auch unter diesen Bedingungen, die Zielzellen (hier Tumorzelle: HCT-8) zu zerstören.

Die Versuche waren so aufgebaut, daß von derartig behandelten PBSZ-Präparaten Aliquots entnommen und mit einer definierten Menge von Tumorzellen kontaminiert wurden. Es konnte gezeigt werden, daß die Tumorzellen selbst bei dieser geringen Konzentration von intakten bsAk, bei der nur ein Teil der Zielantigene besetzt vorliegen, zerstört werden.

Die Auswertung des oben beschriebenen Experiments führte zu folgenden Ergebnissen:

| Patient WaGr Gesamtzellzahl PBSZ: 2,5 x $10^{10}$ + 5 µg bsAk | | | |
|---|---|---|---|
| Aliquot | 5x$10^6$ PBSZ + bsAk + Tumorzellen | | |
| Platte | kein Antikörper | bsAk anti-CD3Xepcam | Tumorzellen/ mononukleäre Zellen (PBSZ)/Loch |
| 24er | 6/6* | 0/6° | 2x$10^4$ / 2x$10^6$ |
| 96er | 12/12 | 0/12 | 5000 / 5 x $10^5$ |
| Tumor-reduktion: | keine | >5log | Tumorzellanteil: 1% °Σ=1,2x$10^5$ Tumorz./ 6-Löcher |

* Anzahl der Löcher mit Tumorwachstum, von 6 bzw. 12 plattierten Löchern, nach 14 Tagen Kultivierung.

° Titrationsversuche mit der Tumorzellinie HCT-8 haben ergeben, daß bereits 1-2 Tumorzellen/Loch ausreichen, um nach 14 Tagen Kultivierung ein deutliches visuell auswertbares Tumorwachstum in 95% aller plattierten Löcher darzustellen.

Berechnung:

Ein intakter bsAk hat ein Molekulargewicht von 150 KDa. D.h. 1 Mol sind 150 Kg und entsprechen definitionsgemäß 6x$10^{23}$ Molekülen. Damit entsprechen 5 pg ca. 2x$10^{13}$ Molekülen.

Nachdem in einem Stammzellpräparat 6,5x$10^9$ T-Zellen bestimmt wurden und eine T-Zelle ca. 30.000 CD3 Moleküle trägt, kommt man auf eine Gesamtzahl von 19,5x$10^{13}$ CD3 Molekülen im Stammzellpräparat.

Da jeder bsAk einen anti-CD3 Bindungsarm besitzt, kann gefolgert werden, daß theoretisch, setzt man die beiden oben berechneten Molekülmengen in Bezug, in diesem konkreten Beispiel nicht mehr als ca. 3000 CD3 Moleküle von den bsAk besetzt werden können.

LITERATUR

1. Haagen et al., Interaction of human moncyte Fcγ receptors with rat IgG2b, J. Immunolog., 1995, 154: 1852-1860

2. Gast G.C., Haagen I.-A., van Houten A.A., Klein S., Duits A.J., de Weger R.A., Vroom T.M., Clark M.R., J. Phillips, van Dijk A.J.G., de Lau W.B.M., Bast B.J.E.G. CD8 T-cell activation after intravenous administration of CD3 X CD19 bispecific antibody in patients with non-Hodgkin lymphoma. Cancer Immunol. Immunother. 40: 390, 1995

3. Tenny, C., Jacobs, S., Stoller, R., Earle, M., and Kirkwood, J. Adoptive cellualr immunotherapy with high-dose chemotherapy and autologous bone marrow rescue (ABMR) for recurrent breast cancer (meeting abstract). Proc. Annu.Meet.Am.Soc.Clin.Oncol; 11: A88, 1992 ISSN: 0736-7589. CO: PMAODO - 7589 CO, 1993.

4. Early Breast Cancer Trialists' Collaborative Group, Systemic treatment of early breast cancer by hormonal, cytotoxic, or immune therapy - 133 randomised trials involving 31 000 recurrences and 24 000 deaths among 75 000 women. Part II Lancet 339: 71-85, 1992

5. Guo et al., Effective tumor vaccines generated by in vitro modification ot tumor cells with cytokines and bispecific monoclonal antibodies. Nature Medicine 3: 451, 1997

6. Lindhofer et al., Preferential species-restricted heavylight chain pairing in rat-mouse quadromas: Implications for a single step purification of bispecific antibodies, J. Immunology 1995, 155:219

## Patentansprüche

1. Verwendung autologer Tumorzellen oder allogener Tumorzellen desselben Tumortyps, die je so behandelt wurden, daß ihr Überleben nach Reinfusion verhindert wird, in zeitlich abgestufter Anwendung mit intakten bispezifischen und/oder trispezifischen Antikörpern mit den nachfolgenden Eigenschaften:

a) Binden an eine T-Zelle;
β) Binden an zumindest ein Antigen auf einer Tumorzelle;
γ) Binden durch ihren Fc-Teil bei bispezifischen Antikörpern oder durch eine dritte Spezifität bei trispezifischen Antikörpern) an Fc-Rezeptor positiven Zellen,

wobei dem zu immunisierenden Menschen zur Herstellung eines Arzneimittels die Tumorzellen und die gegen die Tumorzellen gerichteten intakten Antikörper zeitversetzt verabreicht werden, um eine Immunisierung gegen den Tumor zu erzielen.

**2.** Verwendung nach Anspruch 1,
**dadurch gekennzeichnet,**
**daß** die Tumorzellen vor den Antikörpern oder die Antikörper vor den Tumorzellen verabreicht werden, wobei der Zeitraum zwischen der jeweiligen Verabreichung 1 - 48 Stunden beträgt.

**3.** Verwendung nach Anspruch 1 oder 2,
**dadurch gekennzeichnet,**
**daß** der Zeitraum 1 - 24 Stunden, bevorzugt 1 - 12 Stunden, weiterhin bevorzugt 1 - 6 Stunden oder 1 - 4 Stunden oder 2 - 4 Stunden beträgt.

**4.** Verwendung nach einem oder mehreren der vorhergehenden Ansprüche,
**dadurch gekennzeichnet,**
**daß** die Antikörper in einer Menge, je bezogen auf eine Infusion, von 5 - 500 µg, bevorzugt 10 - 300 µg, weiterhin bevorzugt 10 - 100 µg oder 10 - 50 µg, und die Tumorzellen in einer Menge, bezogen auf eine Infusion, von $10^7$ - $10^9$ Zellen, bevorzugt ca. $10^8$ Zellen, verabreicht werden.

**5.** Verwendung nach einem oder mehreren der vorhergehenden Ansprüche,
**dadurch gekennzeichnet,**
**daß** die Antikörper so ausgewählt werden, daß sie über ihren Fc-Teil bei bispezifischen Antikörpern oder über ihren spezifischen Bindungsarm bei trispezifischen Antikörpern zur Bindung Fc-Rezeptor positiver Zellen befähigt sind, die einen Fcγ-Rezeptor I, II oder III aufweisen.

**6.** Verwendung nach Anspruch 5,
**dadurch gekennzeichnet,**
**daß** die Antikörper zur Bindung an Monozyten, Makrophagen, dendritische Zellen, "Natural Killer"-Zellen (NK-Zellen) und/oder aktivierte neutrophile Zellen als Fcγ-Rezeptor I + III positive Zellen befähigt sind.

**7.** Verwendung nach einem oder mehreren der vorhergehenden Ansprüche,
**dadurch gekennzeichnet,**
**daß** die Antikörper zur Induktion von tumorreaktiven Komplement-bindenden Antikörpern und damit zur Induktion einer humoralen Immunantwort befähigt sind.

**8.** Verwendung nach einem oder mehreren der vorhergehenden Ansprüche,
**dadurch gekennzeichnet,**
**daß** die Antikörper so ausgewählt werden, daß sie über CD2, CD3, CD4, CD5, CD6, CD8, CD28 und/oder CD44 an die T-Zellen binden.

**9.** Verwendung nach einem oder mehreren der vorhergehenden Ansprüche,
**dadurch gekennzeichnet,**
**daß** die Antikörper so ausgewählt werden, daß nach ihrer Bindung an die Fc-Rezeptor positiven Zellen die Expression von CD40, CD80, CD86, ICAM-1 und/oder LFA-3 als kostimulatorische Antigene und/oder die Sekretion von Zytokinen durch die Fc-Rezeptor positive Zelle initiiert oder erhöht wird.

**10.** Verwendung nach Anspruch 9,
**dadurch gekennzeichnet,**
**daß** die Antikörper so ausgewählt werden, daß die Sekretion von IL-1, IL-2, IL-4, IL-6, IL-8, IL-12, INF-γ als Zytokine und/oder von TNF-α erhöht wird.

**11.** Verwendung nach Anspruch 8
**dadurch gekennzeichnet,**
**daß** der Antikörper ein bispezifischer Antikörper ist, der so ausgewählt wird, daß er ein anti-CD3 X anti-Tumor-assoziiertes Antigen- und/oder anti-CD4 X anti-Tumor-assoziiertes Antigen- und/oder anti-CD5 X anti-Tumor-assoziiertes Antigen- und/oder anti-CD6 X anti-Tumor-assoziiertes Antigen- und/oder anti-CD8 X anti-Tumor-assoziiertes Antigen- und/oder anti-CD2 X anti-Tumor-assoziiertes Antigen- und/oder anti-CD28 X anti-Tumor-assoziiertes Antigen- und/oder anti-CD44 X anti-Tumor-assoziiertes Antigen-Antikörper ist.

**12.** Verwendung nach einem oder mehreren der vorhergehenden Ansprüche,
**dadurch gekennzeichnet,**
**daß** der bispezifische Antikörper aus einer oder mehreren der nachfolgenden Isotykombinationen ausgewählt wird:

Ratte-IgG2b/Maus-IgG2a,
Ratte-IgG2b/Maus-IgG2b,
Ratte-IgG2b/Maus-IgG3,

Ratte-IgG2b/Human-IgG1,
Ratte-IgG2b/Human-IgG2,
Ratte-IgG2b/Human-IgG3 orientaler Allotyp G3m(st),
Ratte-IgG2b/Human-IgG4,

Ratte-IgG2b/Ratte-IgG2c,

Maus-IgG2a/Human-IgG3 kaukasische Allotypen G3m(b+g), im folgenden mit * gekennzeichnet]

Maus-IgG2a/Maus-[VH-CH1,VL-CL]-Human-IgG1-[Hinge]-Human-IgG3*-[CH2-CH3]

Maus-IgG2a/Ratte-[VH-CH1,VL-CL]-Human-IgG1-[Hinge]-Human-IgG3*-[CH2-CH3]

Maus-IgG2a/Human-[VH-CH1,VL-CL]-Human-IgG1-[Hinge]-Human-IgG3*-[CH2-CH3]

Maus-[VH-CH1,VL-CL]-Human-IgG1/Ratte-[VH-CH1,VL-CL]-Human-IgG1-[Hinge]-Human-IgG3*-[CH2-CH3]

Maus-[VH-CH1,VL-CL]-Human-IgG4/Ratte-[VH-CH1,VL-CL]-Human-IgG4-[Hinge]-Human-IgG4[N-terminale Region von CH2]-Human-IgG3*[C-terminale Region von CH2: > AS-Position 251]-Human- IgG3*[CH3]

Ratte-IgG2b/Maus-[VH-CH1,VL-CL]-Human-IgG1-[Hinge-CH2-CH3]

Ratte-IgG2b/Maus-[VH-CH1,VL-CL]-Human-IgG2-[Hinge-CH2-CH3]

Ratte-IgG2b/Maus-[VH-CH1,VL-CL]-Human-IgG3-[Hinge-CH2-CH3, orientaler Allotyp]

Ratte-IgG2b/Maus-[VH-CH1,VL-CL]-Human-IgG4-[Hinge-CH2-CH3]

Human-IgG1/Human-[VH-CH1,VL-CL]-Human-IgG1-[Hinge]-Human-IgG3*-[CH2-CH3]

Human-IgG1/Ratte-[VH-CH1,VL-CL]-Human-IgG1-[Hinge]-Human-IgG4[N-terminale Region von CH2]-Human-IgG3*[C-terminale Region von CH2 : > AS-Position 251]-Human-IgG3*[CH3]

Human-IgG1/Maus-[VH-CH1,VL-CL]-Human-IgG1-[Hinge]-Human-IgG4[N-terminale Region von CH2]-Human-IgG3*[C-terminale Region von CH2 : > AS-Position 251]-Human-IgG3*[CH3]

Human-IgG1/Ratte-[VH-CH1,VL-CL]-Human-IgG1-[Hinge]-Human-IgG2[N-terminale Region von CH2]-Human-IgG3*[C-terminale Region von CH2 : > AS-Position 251]-Human-IgG3*[CH3]

Human-IgG1/Maus-[VH-CH1,VL-CL]-Human-IgG1-[Hinge]-Human-IgG2[N-terminale Region von CH2]-Human-IgG3*[C-terminale Region von CH2 : > AS-Position 251]-Human-IgG3*[CH3]

Human-IgG1/Ratte-[VH-CH1,VL-CL]-Human-IgG1-[Hinge]-Human-IgG3*-[CH2-CH3]

Human-IgG1/Maus-[VH-CH1,VL-CL]-Human-IgG1-[Hinge]-Human-IgG3*-[CH2-CH3]

Human-IgG2/Human-[VH-CH1,VL-CL]-Human-IgG2-[Hinge]-Human-IgG3*-[CH2-CH3]

Human-IgG4/Human-[VH-CH1,VL-CL]-Human-IgG4-[Hinge]-Human-IgG3*-[CH2-CH3]

Human-IgG4/Human-[VH-CH1,VL-CL]-Human-IgG4-[Hinge]-Human-IgG4[N-terminale Region von CH2]-Human-IgG3*[C-terminale Region von CH2 : > AS-Position 251]-Human-IgG3*[CH3]

Maus-IgG2b/Ratte-[VH-CH1,VL-CL]-Human-IgG1-[Hinge]-Human-IgG3*-[CH2-CH3]

Maus-IgG2b/Human-[VH-CH1,VL-CL]-Human-IgG1-[Hinge]-Human-IgG3*-[CH2-CH3]

Maus-IgG2b/Maus-[VH-CH1,VL-CL]-Human-IgG1-[Hinge]-Human-IgG3*-[CH2-CH3]

Maus-[VH-CH1,VL-CL]-Human-IgG4/Ratte-[VH-CH1,VL-CL]-Human-IgG4-[Hinge]-Human-IgG4-[CH2]-Human-IgG3*-[CH3]

HumanIgG1/Ratte[VH-CH1,VL-CL]-Human-IgG1[Hinge]-Human-IgG4-[CH2]-HumanIgG3*[CH3]

HumanIgG1/Maus[VH-CH1,VL-CL]-Human-IgG1[Hinge]-Human-IgG4-[CH2]-Human-IgG3*[CH3]

Human-IgG4/Human[VH-CH1,VL-CL]-Human-IgG4-[Hinge]-Human-IgG4-[CH2]-HumanIgG3*-[CH3]

**13.** Verwendung nach einem oder mehreren der vorhergehenden Ansprüche,
**dadurch gekennzeichnet,**
**daß** der bispezifische Antikörper ein heterologer bispezifischer Antikörper, bevorzugt ein heterologer Ratte/Maus bispezifischer Antikörper, ist.

**14.** Verwendung nach Anspruch 8.
**dadurch gekennzeichnet,**
**daß** der Antikörper ein trispezifischer Antikörper ist, der so ausgewählt wird, daß er ein anti-CD3 X anti-Tumor-assoziiertes Antigen- und/oder anti-CD4 X anti-Tumor-assoziiertes Antigen- und/oder anti-CD5 X anti-Tumor-assoziiertes Antigen- und/oder anti-CD6 X anti-Tumor-assoziiertes Antigen- und/oder anti-CD8 X anti-Tumor-assoziiertes Antigen- und/oder anti-CD2 X anti-Tumor-assoziiertes Antigen- und/oder anti-CD28 X anti-Tumor-assoziiertes Antigen- und/oder anti-CD44 X anti-Tumor-assoziiertes Antigen-Antikörper ist.

**15.** Verwendung nach einem oder mehreren der vorhergehenden Ansprüche,
**dadurch gekennzeichnet,**
**daß** Tumorzellen eingesetzt werden, die durch Bestrahlung, bevorzugt durch γ-Bestrahlung, weiterhin bevorzugt in einer Dosisstärke von 50 bis 200 Gy, oder durch chemische Substanzen, bevorzugt Mitomycin C, behandelt wurden.

**16.** Verwendung nach einem oder mehreren der vorhergehenden Ansprüche,
**dadurch gekennzeichnet,**
**daß** der Antikörper so ausgewählt ist, daß er an ein Oberflächenantigen als Zielantigen auf der Tumorzelle bindet, welches induzierbar ist und im nicht induzierten Zustand (Normalzustand) auf der Tumorzelle nicht vorkommt oder in einer derart geringen Zahl, daß die Anzahl für eine Zerstörung der Tumorzelle durch den Antikörper nicht ausreicht.

**17.** Verwendung nach Anspruch 16,
**dadurch gekennzeichnet,**
**daß** zur Steigerung der Immunogenität der Tumorzellen diese vor Verabreichung einer Hitzevorbehandlung unterzogen werden.

**18.** Verwendung nach Anspruch 16 oder 17,

**dadurch gekennzeichnet,**
**daß** als induzierbare Antigene Hitze-Schock-Proteine oder MHC-Klasse-I verwandte MIC-Moleküle eingesetzt werden.

**19.** Verwendung nach einem der Ansprüche 16 bis 18,
**dadurch gekennzeichnet,**
**daß** als Hitze-Schock-Proteine HSP25-, HSP60- oder HSP70-(HSP72-) oder HSP90-Proteine und als MIC-Moleküle MIC-A- und MIC-B-Moleküle eingesetzt werden.

**20.** Verwendung nach Anspruch 19,
**dadurch gekennzeichnet,**
**daß** der Antikörper gegen solche Zielantigene gerichtet ist, die nach Induktion auf der Zielzelle in einer Anzahl von mindestens 100 und höchstens 500.000 pro Zielzelle vorliegen.

**21.** Verwendung nach Anspruch 20,
**dadurch gekennzeichnet,**
**daß** der Antikörper weiterhin so ausgewählt wird, daß er zur Aktivierung Fc-Rezeptor positiver Zellen befähigt ist, wodurch die Expression von Cytokinen und/oder co-stimmulatorischen Antigenen initiiert oder erhöht wird.

**22.** Verwendung nach einem oder mehreren der vorhergehenden Ansprüche,
**dadurch gekennzeichnet,**
**daß** die zeitlich abgestufte Anwendung der intakten bispezifischen und/oder trispezifischen Antikörper zur Verbesserung des Immunisierungserfolges mehrfach erfolgt.

**23.** Pharmazeutische Zusammensetzung als Kombinationspräparat zur zeitlich abgestuften Anwendung von autologen Tumorzellen oder allogenen Tumorzellen desselben Tumortyps, die so behandelt wurden, daß ihr Überleben nach Reinfusion verhindert wird, und intakten bispezifischen und/oder trispezifischen Antikörpern mit den nachfolgenden Eigenschaften:

α) Binden an eine T-Zelle;
β) Binden an zumindest ein Antigen auf einer Tumorzelle;
γ) Binden durch ihren Fc-Teil bei bispezifischen Antikörpern oder durch eine dritte Sepzifität bei trispezifischen Antikörpern an Fc-Rezeptor positiven Zellen;

zur Immunisierung von Menschen gegen den Tumor.

## Claims

**1.** The use of autologous tumor cells or allogeneic tumor cells of the same tumor type each treated to prevent their survival after reinfusion in a time-staggered application with intact bispecific and/or trispecific antibodies having the following properties of:

α) binding to a T cell;
β) binding to at least one antigen on a tumor cell;
γ) binding via their Fc portion in the case of bispecific antibodies or via a third specificity in the case of trispecific antibodies to Fc receptor-positive cells; for the manufacture of a medicament,

wherein the tumor cells and the intact antibodies directed against the tumor cells are administered in a time-staggered manner to the human to be immunized to achieve an immunization against the tumor.

**2.** The use according to claim 1 **characterized in that** said tumor cells are administered prior to the antibodies or said antibodies are administered prior to the tumor cells wherein the interval between each administration is 1 - 48 hours.

**3.** The use according to claim 1 or 2 **characterized in that** the interval is 1 - 24 hours, preferably 1 - 12 hours, further preferred 1 - 6 hours or 1 - 4 hours or 2 - 4 hours.

4. The use according to one or more of the preceding claims **characterized in that** the antibodies are administered in an amount, each based on one infusion, of 5 - 500 µg, preferably 10 - 300 µg, further preferred 1 - 100 µg or 10 - 50 µg, and the tumor cells are administered in an amount, each based on one infusion, of $10^7$ - $10^9$ cells, preferably about $10^8$ cells.

5. The use according to one or more of the preceding claims, **characterized in that** said antibodies are selected to be capable of binding, via their Fc portion in the case of bispecific antibodies or via their specific binding arm in the case of trispecific antibodies to Fc receptor-positive cells having an Fcγ receptor I, II, or III.

6. The use according to claim 5, **characterized in that** said antibodies are able to bind to monocytes, makrophages, dendritic cells, "natural killer" cells (NK cells) and/or activated neutrophils being Fcγ receptor I-positive cells.

7. The use according to one or more of the preceding claims, **characterized in that** said antibodies are capable of inducing tumor-reactive complement-binding antibodies and therefore of inducing a humoral immune response.

8. The use according to one or more of the preceding claims, **characterized in that** said antibodies are selected to bind to the T cells via CD2, CD3, CD4, CD5, CD6, CD8, CD28, and/or CD44.

9. The use according to one or more of the preceding claims, **characterized in that** said antibodies are selected so that following their binding to the Fc receptor-positive cells the expression of CD40, CD80, CD86, ICAM-1, and/or LFA-3 being co-stimulatory antigens and/or the secretion of cytokins by the Fc receptor-positive cell is initiated or increased.

10. The use according to claim 9, **characterized in that** the antibodies are selected so that the secretion of IL-1, IL-2, IL-4, IL-6, IL-8, IL-12, INF-γ being cytokins and/or of TNF-α is increased.

11. The use according to claim 8, **characterized in that** said antibody is a bispecific antibody, which is selected to be an anti-CD3 X anti-tumor-associated antigen antibody and/or anti-CD4 X anti-tumor-associated antigen antibody and/or anti-CD5 X anti-tumor-associated antigen antibody and/or anti-CD6 X anti-tumor-associated antigen antibody and/or anti-CD8 X anti-tumor-associated antigen antibody and/or anti-CD2 X anti-tumor-associated antigen antibody and/or anti-CD28 X anti-tumor-associated antigen antibody and/or anti-CD44 X anti-tumor-associated antigen antibody.

12. The use according to one or more of the preceding claims, **characterized in that** said bispecific antibody is selected from one or more of the following combinations of isotypes:

rat-IgG2b/mouse-IgG2a,
rat-IgG2b/mouse-IgG2b,
rat-IgG2b/mouse-IgG3,

rat-IgG2b/human-IgG1,
rat-IgG2b/human-IgG2,
rat-IgG2b/human-IgG3 oriental allotype G3m(st)
rat-IgG2b/human-IgG4,

rat-IgG2b/rat-IgG2c,

mouse-IgG2a/human-IgG3 caucasian allotypes G3m(b+g), in the following indicated as *

mouse-IgG2a/mouse-[VH-CH1,VL-CL]-human-IgG1-[hinge]-human-IgG3*-[CH2-CH3]

mouse-IgG2a/rat-[VH-CH1,VL-CL]-human-IgG1-[hinge]-human- IgG3*-[CH2-CH3]

mouse-IgG2a/human-[VH-CH1,VL-CL]-human-IgG1-[hinge]-human- IgG3*-[CH2-CH3]

mouse-[VH-CH1,VL-CL]-human-IgG1/rat-[VH-CH1,VL-CL]-human-IgG1-[hinge]-human-IgG3*-[CH2-CH3]

mouse-[VH-CH 1,VL-CL]-human-IgG4/rat-[VH-CH1,VL-CL]-human-IgG4-[hinge]-human-IgG4[N-terminal region of CH2]-human- IgG3*[C-terminal region of CH2: > aa position 251]-human- IgG3*[CH3]

rat-IgG2b/mouse-[VH-CH1,VL-CL]-human-IgG1-[hinge-CH2-CH3]

rat-IgG2b/mouse-[VH-CH1,VL-CL]-human-IgG2-[hinge-CH2-CH3]

rat-IgG2b/mouse-[VH-CH1,VL-CL]-human-IgG3-[hinge-CH2-CH3, oriental allotype]

rat-IgG2b/mouse-[VH-CH1,VL-CL]-human-IgG4-[hinge-CH2-CH3]

human-IgG1/human-[VH-CH1,VL-CL]-human-IgG1-[hinge]-human-IgG3 *-[CH2-CH3]

human-IgG1/rat-[VH-CH1,VL-CL]-human-IgG1-[hinge]-human-IgG4[N-terminal region of CH2]-human-IgG3* [C-terminal region of CH2 : > aa position 251]-human-IgG3*[CH3]

human-IgG1/mouse-[VH-CH1,VL-CL]-human-IgG1-[hinge]-human-IgG4[N-terminal region of CH2]-human-IgG3*[C-terminal region of CH2 : > aa position 251]-human-IgG3*[CH3]

human-IgG1/rat-[VH-CH1,VL-CL]-human-IgG1-[hinge]-human-IgG2[N-terminal region of CH2]-human-IgG3* [C-terminal region of CH2 : > aa position 251]-human-IgG3*[CH3]

human-IgG1/mouse-[VH-CH1,VL-CL]-human-IgG1-[hinge]-human-IgG2[N-terminal region of CH2]-human-IgG3*[C-terminal region of CH2 : > aa position 251]-human-IgG3*[CH3]

human-IgG1/rat-[VH-CH1,VL-CL]-human-IgG1-[hinge]-human-IgG3*-[CH2-CH3]

human-IgG1/mouse-[VH-CH1,VL-CL]-human-IgG1-[hinge]-human-IgG3*-[CH2-CH3]

human-IgG2/human-[VH-CH1,VL-CL]-human-IgG2-[hinge]-human-IgG3*-[CH2-CH3]

human-IgG4/human-[VH-CH1,VL-CL]-human-IgG4-[hinge]-human-IgG3*-[CH2-CH3]

human-IgG4/human-[VH-CH1,VL-CL]-human-IgG4-[hinge]-human-IgG4[N-terminal region of CH2]-human-IgG3*[C-terminal region of CH2 : > aa position 251]-human-IgG3*[CH3]

mouse-IgG2b/rat-[VH-CH1,VL-CL]-human-IgG1-[hinge]-human-IgG3*-[CH2-CH3]

mouse-IgG2b/human-[VH-CH1,VL-CL]-human-IgG1-[hinge]-human-IgG3*-[CH2-CH3]

mouse-IgG2b/mouse-[VH-CH1,VL-CL]-human-IgG1-[hinge]-human-IgG3*-[CH2-CH3]

mouse-[VH-CH1,VL-CL]-human-IgG4/rat-[VH-CH1,VL-CL]-human-IgG4-[hinge]-human-IgG4-[CH2]-human-IgG3*-[CH3]

human-IgG1/rat-[VH-CH1,VL-CL]-human-IgG1-[hinge]-human-IgG4-[CH2]-human-IgG3*-[CH3]

human-IgG1/mouse-[VH-CH1,VL-CL]-human-IgG1-[hinge]-human-IgG4-[CH2]-human-IgG3*-[CH3]

human-IgG4/human-[VH-CH1,VL-CL]-human-IgG4-[hinge]-human-IgG4-[CH2]-human-IgG3*-[CH3].

**13.** The use according to one or more of the preceding claims, **characterized in that** said bispecific antibody is a heterologous bispecific antibody, preferably a heterologous rat/mouse bispecific antibody.

**14.** The use according to claim 8, **characterized in that** said antibody is a trispecific antibody, which is selected to be an anti-CD3 X anti-tumor-associated antigen antibody and/or anti-CD4 X anti-tumor-associated antigen antibody and/or anti-CD5 X anti-tumor-associated antigen antibody and/or anti-CD6 X anti-tumor-associated antigen antibody and/or anti-CD8 X anti-tumor-associated antigen antibody and/or anti-CD2 X anti-tumor-associated antigen antibody and/or anti-CD28 X anti-tumor-associated antigen antibody and/or anti-CD44 X anti-tumor-associated antigen antibody.

**15.** The use according to one or more of the preceding claims, **characterized in that** tumor cells are used which have been treated by irradiation, preferably by gamma-irradiation, further preferred in a dose of 50 to 200 Gy, or by chemical substances, preferably mitomycin C.

**16.** The use according to one or more of the preceding claims, **characterized in that** said antibody is selected to bind to a surface antigen being the target antigen on the tumor cell which is inducible and which is absent form the tumor cell in the uninduced state (normal state) or is present in an amount which is so low that the number is insufficient for destruction of the tumor cell by the antibody.

**17.** The use according to claim 16, **characterized in that** the tumor cells are subjected to a heat pretreatment to increase the immunogenicity.

**18.** The use according to claim 16 or 17, **characterized in that** heat shock proteins or MHC class I-related MIC molecules are employed as the inducible antigens.

**19.** The use according to any of the claims 16 to 18, **characterized in that** HSP25, Hsp60 or Hsp70 (Hsp72) or Hsp90 proteins are used as heat shock proteins and MIC A and MIC B molecules are used as MIC molecules.

**20.** The use according to claim 19, **characterized in that** said antibody is directed against target antigens which after induction of the target cell are present in an amount of at least 100 and at the most 500,000 per target cell.

**21.** The use according to claim 20, **characterized in that** the antibody is further selected to be capable of activating Fc receptor-positive cells whereby the expression of cytokins and/or co-stimulatory antigens is initiated or increased.

**22.** The use according to one or more of the preceding claims, **characterized in that** the time-staggered application of the intact bispecific and/or trispecific antibodies is performed several times to increase the success of the immunization.

**23.** Pharmaceutical preparation in form of a combination preparation for a time-staggered application of autologous tumor cells orallogeneic tumor cells of the same tumor, each treated to prevent their survival after thereinfusion, and intact bispecific and/or trispecific antibodies having the following properties of:

α) binding to a T-cell
β) binding to at least one antigen on a tumor cell;
γ) binding via their Fc portion in the case of bispecific antibodies or via a third specificity in the case of trispecific antibodies to Fc receptor-positive cells;

for immunization of humans against the tumor.

## Revendications

**1.** Utilisation de cellules tumorales autologues ou de cellules tumorales allogéniques du même type de tumeur, qui ont été traitées chacune de manière à empêcher leur survie après reperfusion, lesquelles sont administrées de manière graduée dans le temps avec des anticorps bispécifiques et/ou trispécifiques intacts présentant les propriétés suivantes :

α) liaison à une cellule T ;
β) liaison à au moins un antigène sur une cellule tumorale ;
γ) liaison des cellules positives au récepteur Fc par leur partie Fc, dans le cas d'anticorps bispécifiques, ou par une troisième spécificité, dans le cas d'anticorps trispécifiques,

pour la fabrication d'un médicament, les cellules tumorales et les anticorps intacts dirigés contre les cellules tumorales étant administrés de manière décalée dans le temps au patient humain à immuniser, en vue d'obtenir une immunisation contre la tumeur.

2. Utilisation selon la revendication 1, **caractérisée en ce que** les cellules tumorales sont administrées avant les anticorps ou les anticorps sont administrés avant les cellules tumorales, l'intervalle de temps entre chaque administration étant de l'ordre de 1 à 48 heures.

3. Utilisation selon la revendication 1 ou 2, **caractérisée en ce que** l'intervalle de temps est de l'ordre de 1 à 24 heures, de préférence de 1 à 12 heures, de manière encore plus préférée de 1 à 6 heures ou de 1 à 4 heures ou 2 à 4 heures.

4. Utilisation selon l'une ou plusieurs des revendications précédentes, **caractérisée en ce que** la dose d'anticorps administrée à chaque injection est comprise entre 5 et 500 µg, de préférence entre 10 et 300 µg, de manière encore plus préférée entre 10 et 100 µg ou entre 10 et 50 µg, et la dose de cellules tumorales administrée à chaque injection est comprise entre $10^7$ et $10^9$ cellules, de préférence environ $10^8$ cellules.

5. Utilisation selon l'une ou plusieurs des revendications précédentes, **caractérisée en ce que** les anticorps sont choisis de telle sorte que, par l'intermédiaire de leur partie Fc, dans le cas des anticorps bispécifiques, ou par l'intermédiaire de leur bras de liaison spécifique, dans le cas des anticorps trispécifiques, ils sont aptes à se lier à des cellules positives au récepteur Fc, qui contiennent un récepteur Fcγ I, II ou III.

6. Utilisation selon la revendication 5, **caractérisée en ce que** les anticorps sont aptes à se lier à des monocytes, des macrophages, des cellules dendritiques, des cellules NK dites « Natural Killer » et/ou des cellules neutrophiles activées, en tant que cellules positives au récepteur Fcγ I + III.

7. Utilisation selon l'une ou plusieurs des revendications précédentes, **caractérisée en ce que** les anticorps sont aptes à induire des anticorps de liaison au complément réactifs vis-à-vis de la tumeur et, de ce fait, à induire une réponse immunitaire humorale.

8. Utilisation selon l'une ou plusieurs des revendications précédentes, **caractérisée en ce que** les anticorps sont choisis de telle sorte qu'ils se lient aux cellules T par l'intermédiaire de CD2, CD3, CD4, CD5, CD6, CD8, CD28 et/ou CD44.

9. Utilisation selon l'une ou plusieurs des revendications précédentes, **caractérisée en ce que** les anticorps sont choisis de telle sorte que, après leur liaison aux cellules positives au récepteur Fc, l'expression de CD40, CD80, CD86, ICAM-1 et/ou LFA-3 en tant qu'antigènes co-stimulatoires et/ou la sécrétion des cytocines est initiée ou accrue par les cellules positives au récepteur Fc.

10. Utilisation selon la revendication 9, **caractérisée en ce que** les anticorps sont choisis de telle sorte que la sécrétion de IL-1, IL-2, IL-4, IL-6, IL-8, IL-12, INF-γ en tant que cytocine et/ou de TNF-α est accrue.

11. Utilisation selon la revendication 8, **caractérisée en ce que** l'anticorps est un anticorps bispécifique, qui est choisi de telle sorte qu'il est un anticorps anti-CD3 X anti-antigène associé à une tumeur et/ou un anticorps anti-CD4 X anti-antigène associé à une tumeur et/ou un anticorps anti-CD5 X anti-antigène associé à une tumeur et/ou un anticorps anti-CD6 X anti-antigène associé à une tumeur et/ou un anticorps anti-CD8 X anti-antigène associé à une tumeur et/ou un anticorps anti-CD2 X anti-antigène associé à une tumeur et/ou un anticorps anti-CD28 X anti-antigène associé à une tumeur et/ou un anticorps anti-CD44 X anti-antigène associé à une tumeur.

12. Utilisation selon l'une ou plusieurs des revendications précédentes, **caractérisée en ce que** l'anticorps bispécifique est choisi parmi une ou plusieurs des isoty-combinaisons suivantes :

rat-IgG2b/souris-IgG2a,
rat-IgG2b/souris-IgG2b,
rat-IgG2b/souris-IgG3,

rat-IgG2b/humain-IgG1,
rat-IgG2b/humain-IgG2,
rat-IgG2b/humain-IgG3 allotype oriental G3m(st)
rat-IgG2b/humain-IgG4,

rat-IgG2b/rat-IgG2c,

souris-IgG2a/humain-IgG3 allotypes caucasiens G3m(b+g), désignés ci-après par *,

souris-IgG2a/souris-[VH-CH1, VL-CL]-humain-IgG1-[charnière]-humain-IgG3*-[CH2-CH3]

souris-IgG2a/rat-[VH-CH1, VL-CL]-humain-IgG1-[charnière]-humain-IgG3*-[CH2-CH3]

souris-IgG2a/humain-[VH-CH1, VL-CL]-humain-IgG1-[charnière]-humain-IgG3*-[CH2-CH3]

souris-[VH-CH1, VL-CL]-humain-IgG1/rat-[VH-CH1, VL-CL]- humain-IgG1-[charnière]-humain-IgG3*-[CH2-CH3]

souris-[VH-CH1, VL-CL]-humain-IgG4/rat-[VH-CH1, VL-CL]- humain-IgG4-[charnière]-humain-IgG4[région N-terminale de CH2]-humain-IgG3 *[région C-terminale de CH2 : > AS-position 251]-humain- IgG3*[CH3]

rat-IgG2b/souris-[VH-CH1, VL-CL]-humain-IgG1-[charnière-CH2-CH3]

rat-IgG2b/souris-[VH-CH1, VL-CL]-humain-IgG2-[charnière-CH2-CH3]

rat-IgG2b/souris-[VH-CH1, VL-CL]-humain-IgG3-[charnière-CH2-CH3, allotype oriental]

rat-IgG2b/souris-[VH-CH1, VL-CL]-humain-IgG4-[charnière-CH2-CH3]

humain-IgG1/humain-[VH-CH1, VL-CL]-humain-IgG1-[charnière]-humain-IgG3*-[CH2-CH3]

humain-IgG1/rat-[VH-CH1, VL-CL]- humain-IgG1-[charnière]-humain-IgG4[région N-terminale de CH2]-humain-IgG3*[C-région terminale de CH2 : > AS-position 251]-humain- IgG3*[CH3]

humain-IgG1/souris-[VH-CH1, VL-CL]- humain-IgG1-[charnière]-humain-IgG4[région N-terminale de CH2]-humain-IgG3*[région C-terminale de CH2 : > AS-position 251]-humain- IgG3*[CH3]

humain-IgG1/rat-[VH-CH1, VL-CL]- humain-IgG1-[charnière]-humain-IgG2[région N-terminale de CH2]-humain-IgG3*[région C-terminale de CH2 : > AS-position 251]-humain- IgG3*[CH3]

humain-IgG1/souris-[VH-CH1, VL-CL]- humain-IgG1-[charnière]-humain-IgG2[région N-terminale de CH2]-humain-IgG3*[région C-terminale de CH2 : > AS-position 251]-humain- IgG3*[CH3]

humain-IgG1/rat-[VH-CH1, VL-CL]-humain-IgG1-[charnière]-humain-IgG3*-[CH2-CH3]

humain-IgG1/souris-[VH-CH1, VL-CL]-humain-IgG1-[charnière]-humain-IgG3*-[CH2-CH3]

humain-IgG2/humain-[VH-CH1, VL-CL]-humain-IgG2-[charnière]-humain-IgG3*-[CH2-CH3]

humain-IgG4/humain-[VH-CH1, VL-CL]-humain-IgG4-[charnière]-humain-IgG3*-[CH2-CH3]

humain-IgG4/humain-[VH-CH1, VL-CL]- humain-IgG4-[charnière]-humain-IgG4[région N-terminale de CH2]-humain-IgG3*[région C-terminale de CH2 : > AS-position 251]-humain- IgG3*[CH3]

souris-IgG2b/rat-[VH-CH1, VL-CL]-humain-IgG1-[charnière]-humain-IgG3*-[CH2-CH3]

souris-IgG2b/humain-[VH-CH1, VL-CL]-humain-IgG1-[charnière]-humain-IgG3*-[CH2-CH3]

souris-IgG2b/souris-[VH-CH1, VL-CL]-humain-IgG1-[charnière]-humain-IgG3*-[CH2-CH3]

souris-[VH-CH1, VL-CL]-humain-IgG4/rat-[VH-CH1, VL-CL]- humain-IgG4-[charnière]-humain-IgG4-[CH2]-humain-IgG3 *-[CH3]

humain-IgG1/rat-[VH-CH1, VL-CL]-humain-IgG1-[charnière]-humain-IgG4-[CH2]-humain-IgG3 *-[CH3]

humain-IgG1/souris-[VH-CH1, VL-CL]-humain-IgG1-[charnière]-humain-IgG4-[CH2]-humain-IgG3*-[CH3]

humain-IgG4/humain-[VH-CH1, VL-CL]-humain-IgG4-[charnière]-humain-IgG4-[CH2]-humain-IgG3*-[CH3].

**13.** Utilisation selon l'une ou plusieurs des revendications précédentes, **caractérisée en ce que** l'anticorps bispécifique est un anticorps bispécifique hétérologue, de préférence un anticorps bispécifique hétérologue rat/souris.

**14.** Utilisation selon la revendication 8, **caractérisée en ce que** l'anticorps est un anticorps trispécifique, qui est choisi de telle sorte qu'il est un anticorps anti-CD3 X anti-antigène associé à une tumeur et/ou un anticorps anti-CD4 X anti-antigène associé à une tumeur et/ou un anticorps anti-CD5 X anti-antigène associé à une tumeur et/ou un anticorps anti-CD6 X anti-antigène associé à une tumeur et/ou un anticorps anti-CD8 X anti-antigène associé à une tumeur et/ou un anticorps anti-CD2 X anti-antigène associé à une tumeur et/ou un anticorps anti-CD28 X anti-antigène associé à une anti-tumeur et/ou un anticorps anti-CD44 X anti-antigène associé à une tumeur.

**15.** Utilisation selon l'une ou plusieurs des revendications précédentes, **caractérisée en ce que** l'on utilise des cellules tumorales qui ont été traitées par radiation, de préférence par radiation γ, de manière encore plus préférée avec une dose de radiation absorbée de l'ordre de 50 à 200 Gy, ou par des substances chimiques, de préférence la mitomycine C.

**16.** Utilisation selon l'une ou plusieurs des revendications précédentes, **caractérisée en ce que** l'anticorps est choisi de telle sorte qu'il se lie à un antigène de surface en tant qu'antigène cible sur la cellule tumorale, lequel peut être induit et n'apparaît pas à l'état non induit (état normal) sur la cellule tumorale ou dans un nombre si faible qu'il ne suffit pas pour une détérioration de la cellule tumorale par l'anticorps.

**17.** Utilisation selon la revendication 16, **caractérisée en ce que** pour accroître l'immunogénéité des cellules tumorales, celles-ci sont soumises à un traitement thermique avant leur administration.

**18.** Utilisation selon la revendication 16 ou 17, **caractérisée en ce que** les antigènes à induire utilisés sont des protéines de choc thermique ou des molécules MIC apparentées aux molécules de classe I du CMH.

**19.** Utilisation selon l'une ou plusieurs des revendications 16 à 18, **caractérisée en ce que** les protéines de choc thermique utilisées sont des protéines HSP25, HSP60 ou HSP70 (protéine HSP72) ou HSP90 et les molécules MIC utilisées sont des molécules MIC A et des molécules MIC B.

**20.** Utilisation selon la revendication 19, **caractérisée en ce que** l'anticorps est dirigé contre des antigènes cibles qui, après induction, sont présents sur la cellule cible à raison de minimum 100 et maximum 500 000 par cellule cible.

**21.** Utilisation selon la revendication 20, **caractérisée en ce que** l'anticorps est choisi, par ailleurs, de telle sorte qu'il est apte à activer les cellules positives au récepteur Fc, moyennant quoi l'expression des cytocines et/ou des antigènes co-stimulatoires est initiée ou accrue.

**22.** Utilisation selon l'une ou plusieurs des revendications précédentes, **caractérisée en ce que** l'administration graduée dans le temps des anticorps bispécifiques et/ou trispécifiques intacts est répétée plusieurs fois pour améliorer le résultat d'immunisation.

**23.** Composition pharmaceutique sous forme de préparation combinée destinée à l'administration graduée dans le temps de cellules tumorales autologues ou de cellules tumorales allogéniques du même type de tumeur, qui ont été traitées chacune de manière à empêcher leur survie après reperfusion, et d'anticorps bispécifiques et/ou trispécifiques intacts possédant les propriétés suivantes :

α) liaison à une cellule T ;
β) liaison à au moins un antigène sur une cellule tumorale ;
γ) liaison des cellules positives au récepteur Fc par leur partie Fc, dans le cas d'anticorps bispécifiques, ou par une troisième spécificité, dans le cas d'anticorps trispécifiques,

destinée à immuniser des patients humains contre la tumeur.

# Abb. 1

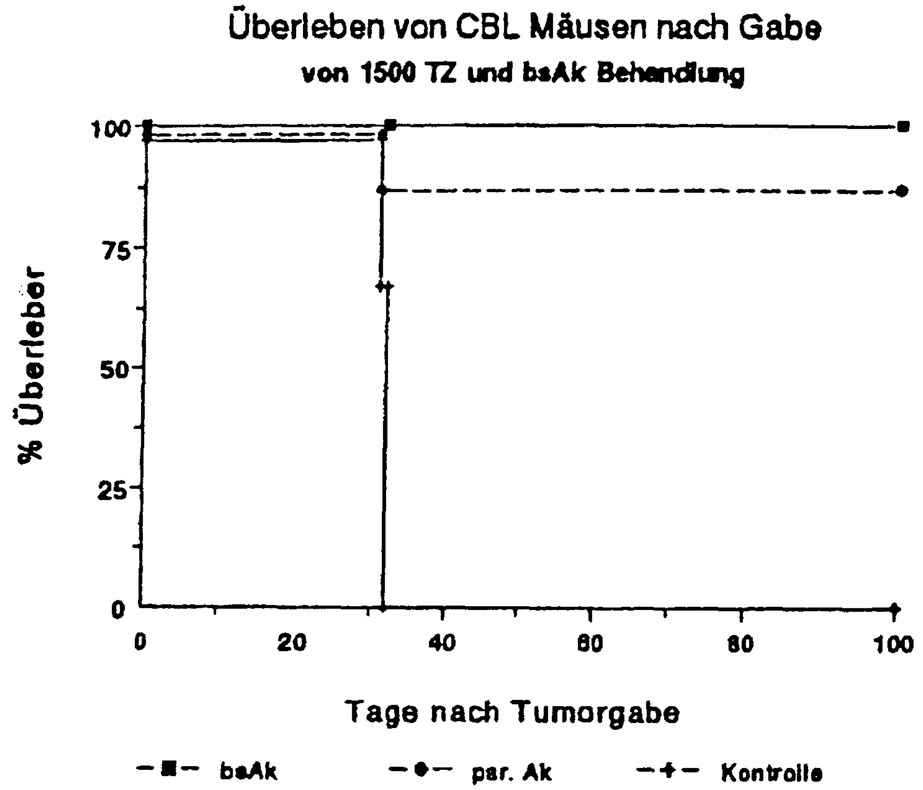
Überleben von CBL Mäusen nach Gabe
von 1500 TZ und bsAk Behandlung
% Überleber
100
75
50
25
0
0
20
40
60
80
100
Tage nach Tumorgabe
bsAk
par. Ak
Kontrolle

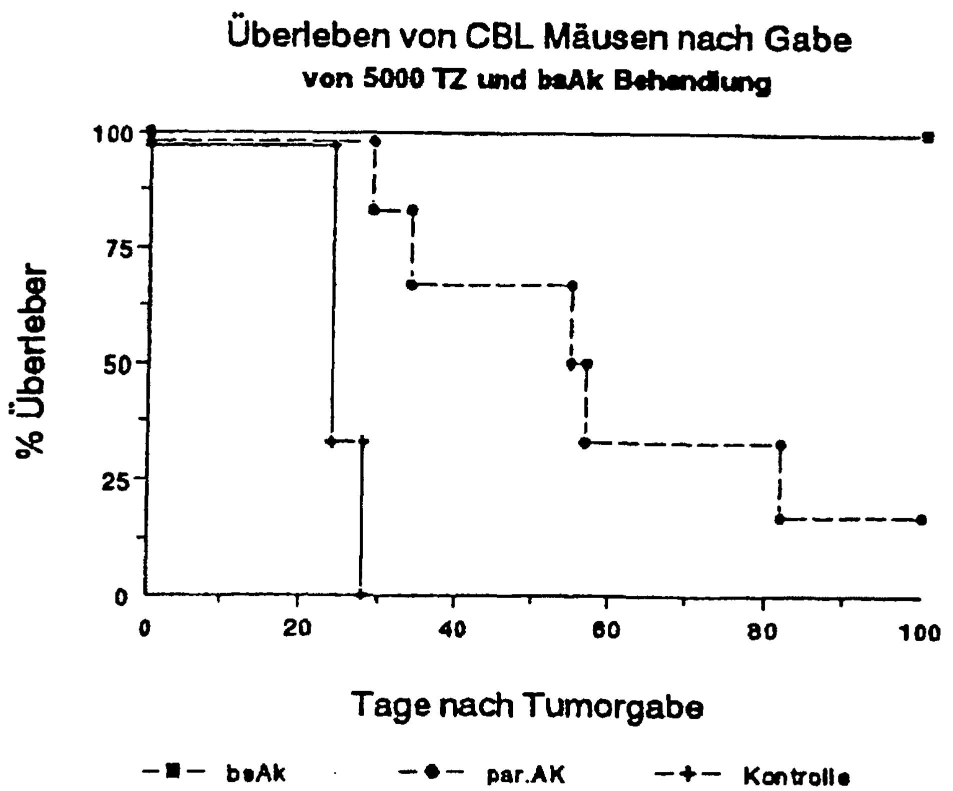
Überleben von CBL Mäusen nach Gabe
von 5000 TZ und bsAk Behandlung
% Überleber
100
75
50
25
0
0
20
40
60
80
100
Tage nach Tumorgabe
bsAk
par.AK
Kontrolle

# Abb. 2

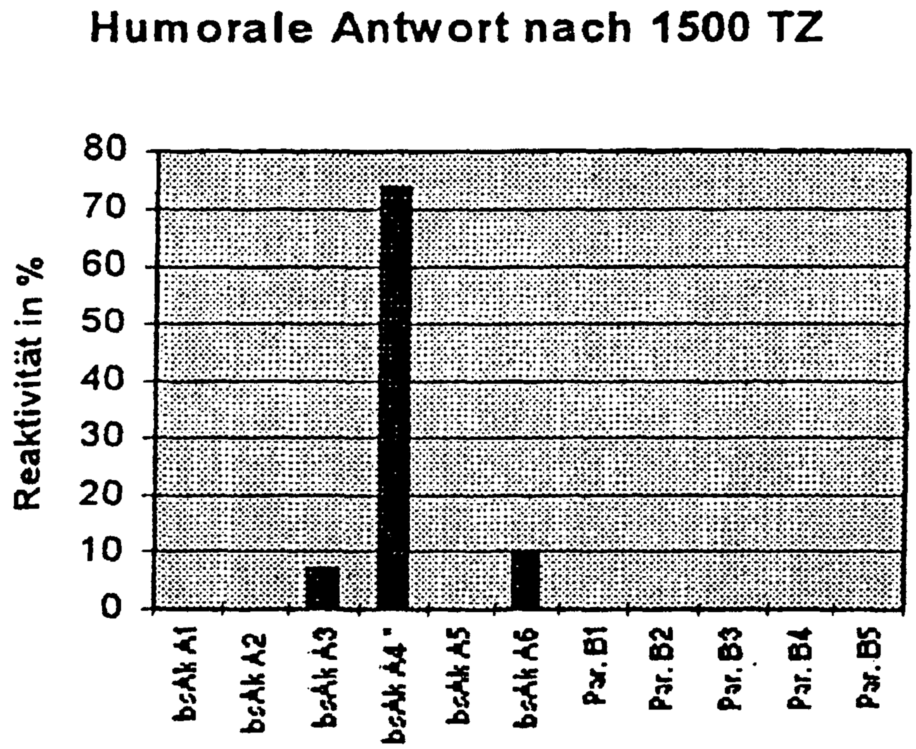
Humorale Antwort nach 1500 TZ
Reaktivität in %
80
70
60
50
40
30
20
10
0
bsAk A1
bsAk A2
bsAk A3
bsAk A4*
bsAk A5
bsAk A6
Par. B1
Par. B2
Par. B3
Par. B4
Par. B5

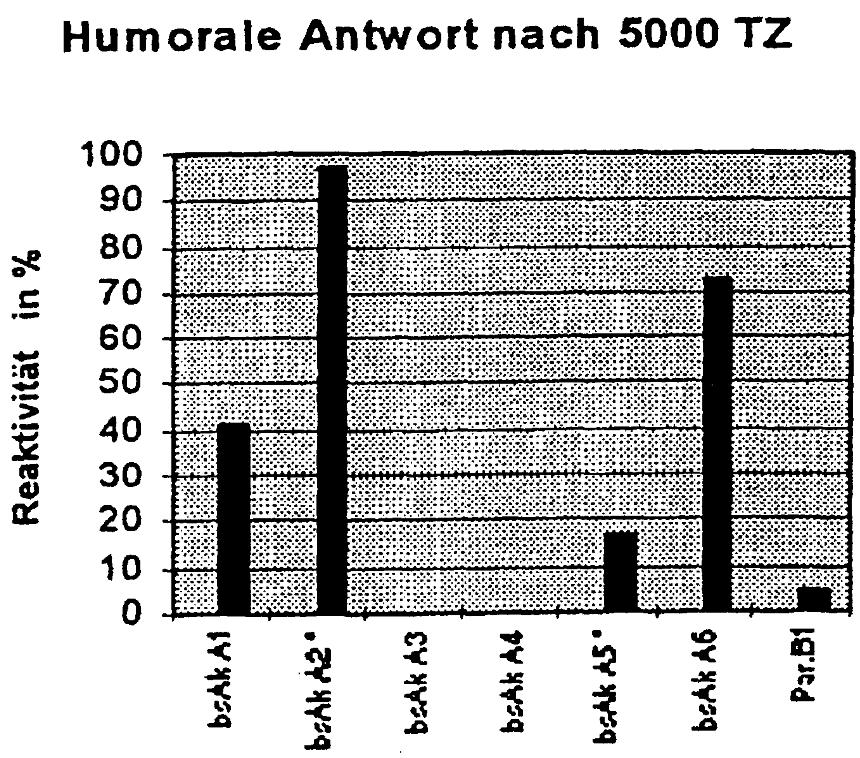
Humorale Antwort nach 5000 TZ
Reaktivität in %
100
90
80
70
60
50
40
30
20
10
0
bsAk A1
bsAk A2*
bsAk A3
bsAk A4
bsAk A5*
bsAk A6
Par.B1

Abb. 3 A

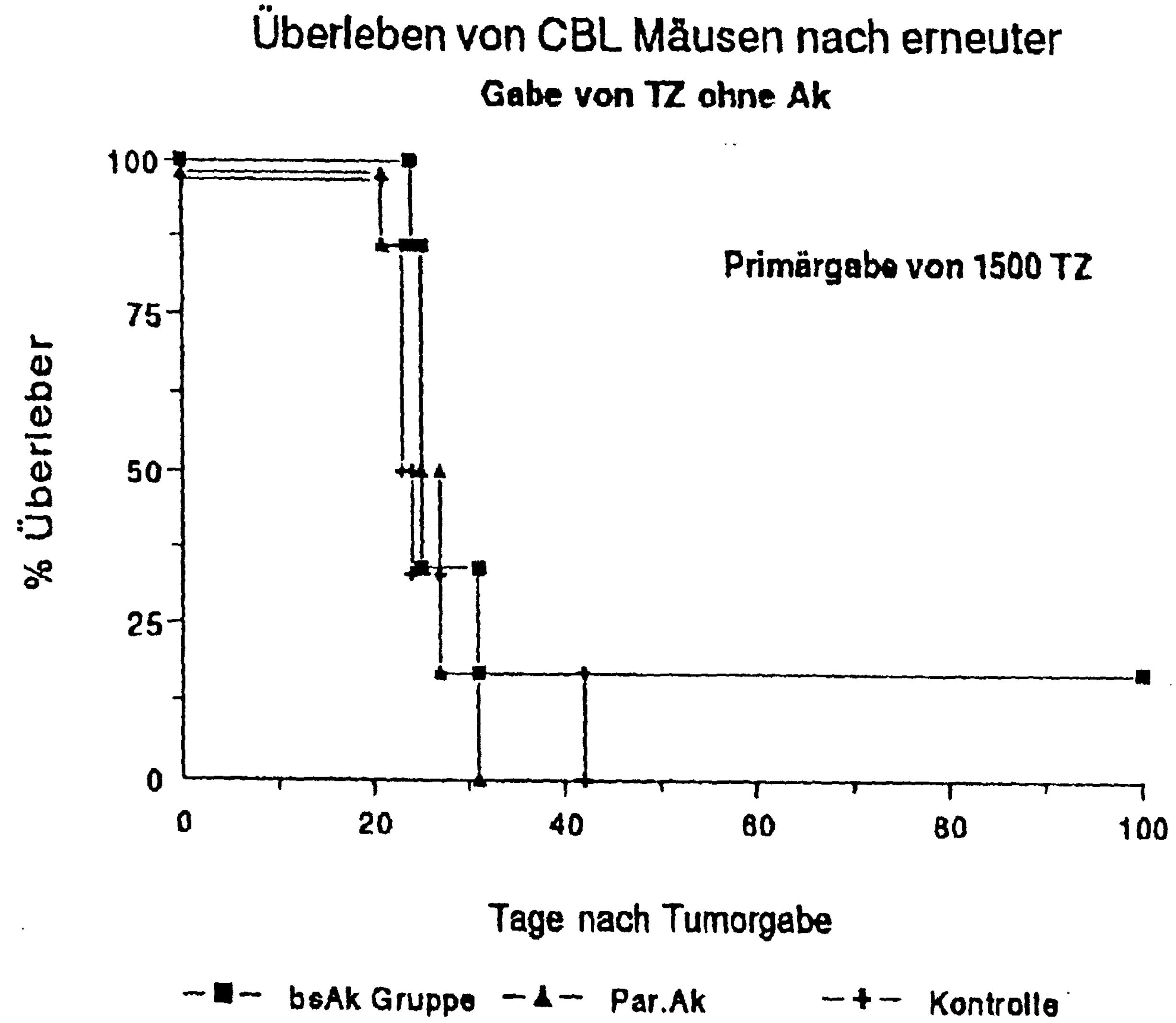
Überleben von CBL Mäusen nach erneuter
Gabe von TZ ohne Ak
Primärgabe von 1500 TZ
% Überleber
100
75
50
25
0
0
20
40
60
80
100
Tage nach Tumorgabe
bsAk Gruppe
Par.Ak
Kontrolle

Abb. 3B

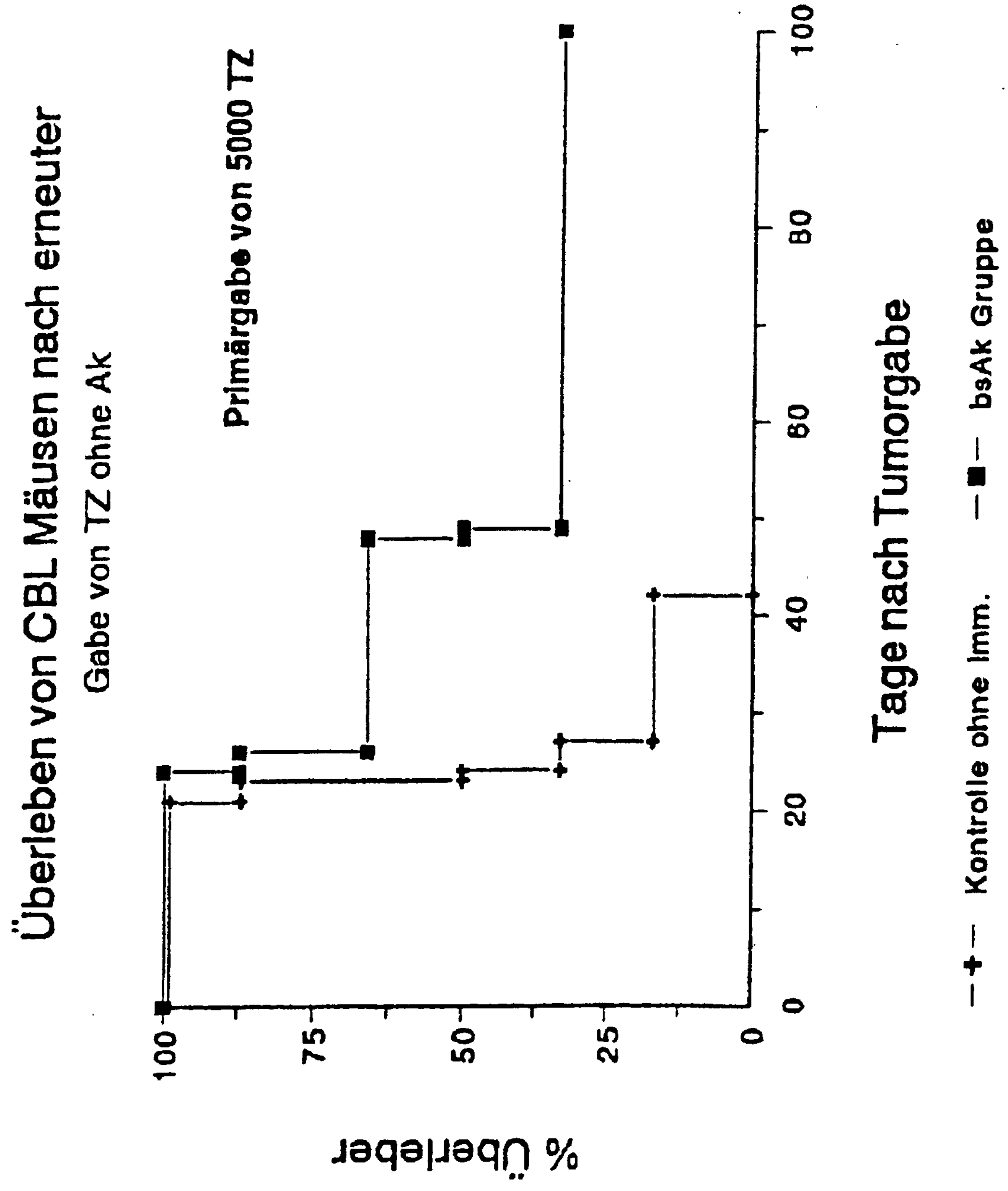
Überleben von CBL Mäusen nach erneuter
Gabe von TZ ohne Ak
Primärgabe von 5000 TZ
% Überleber
100
75
50
25
0
0
20
40
60
80
100
Tage nach Tumorgabe
Kontrolle ohne Imm.
bsAk Gruppe

Abb.4
Die Rolle von akzessorischen Zellen bei der Tumor-Immuntherapie mittels bispezifischer Antikörper

A

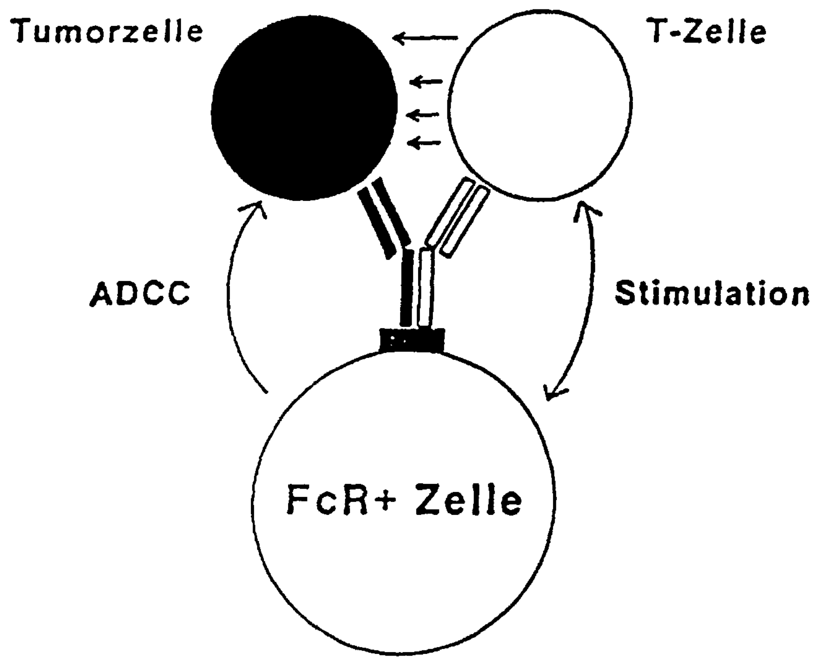


B

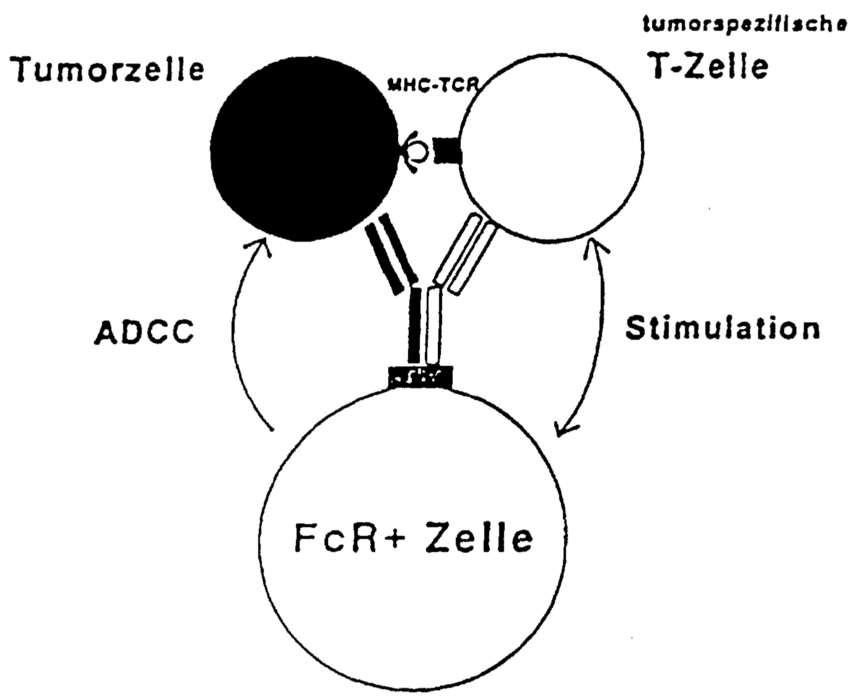

ABBILDUNG 5

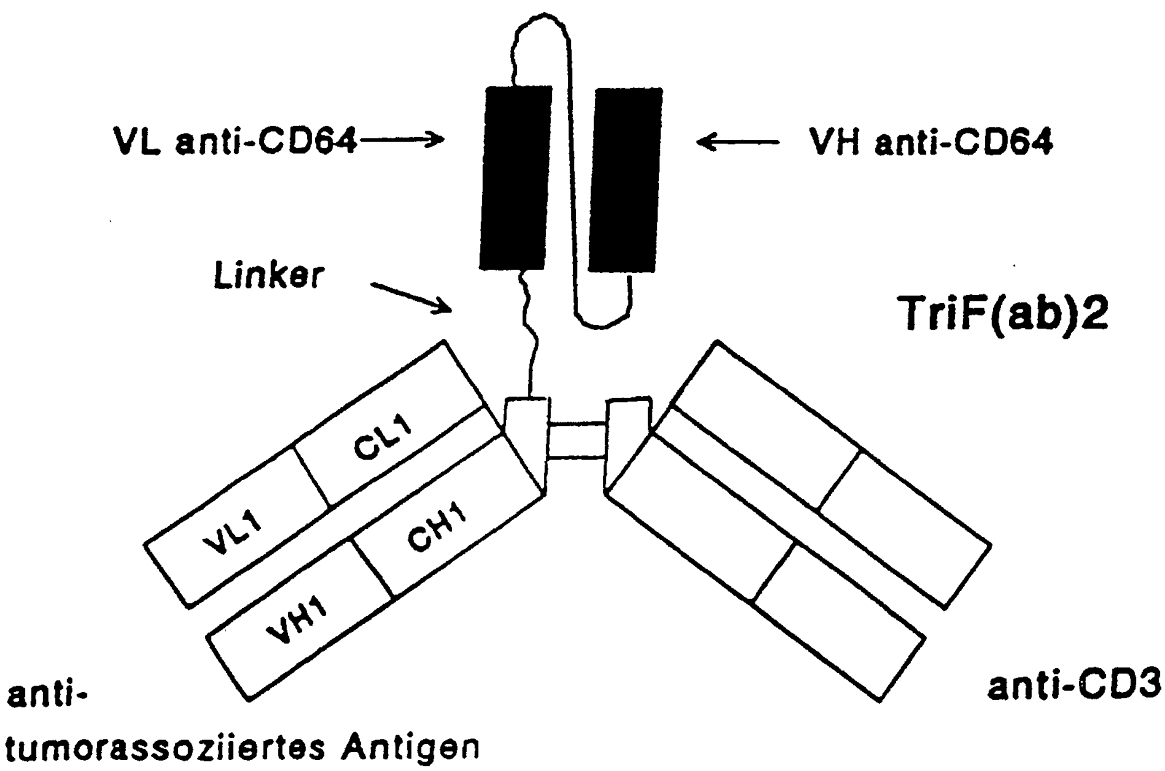
VL anti-CD64
VH anti-CD64
Linker
TriF(ab)2
CL1
VL1
CH1
VH1
anti-
tumorassoziiertes Antigen
anti-CD3

ABBILDUNG 6

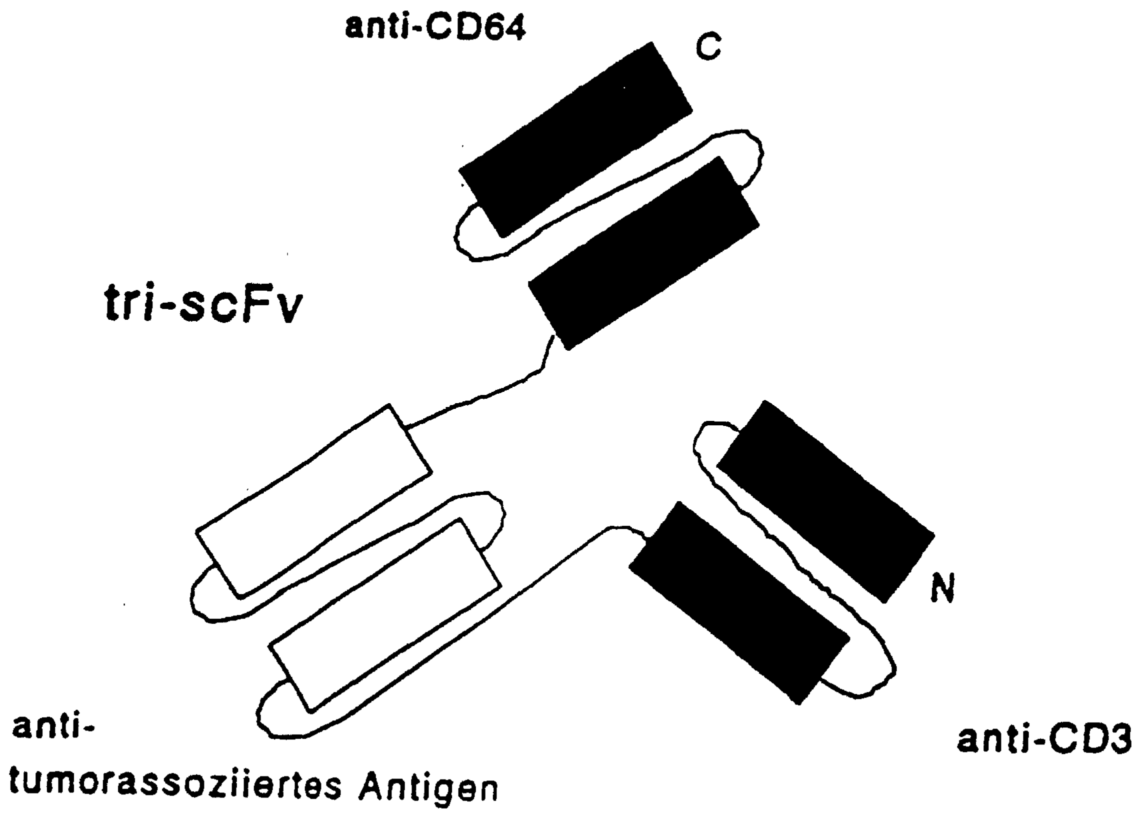
anti-CD64
C
tri-scFv
N
anti-
tumorassoziiertes Antigen
anti-CD3

Abb. 7 A + B

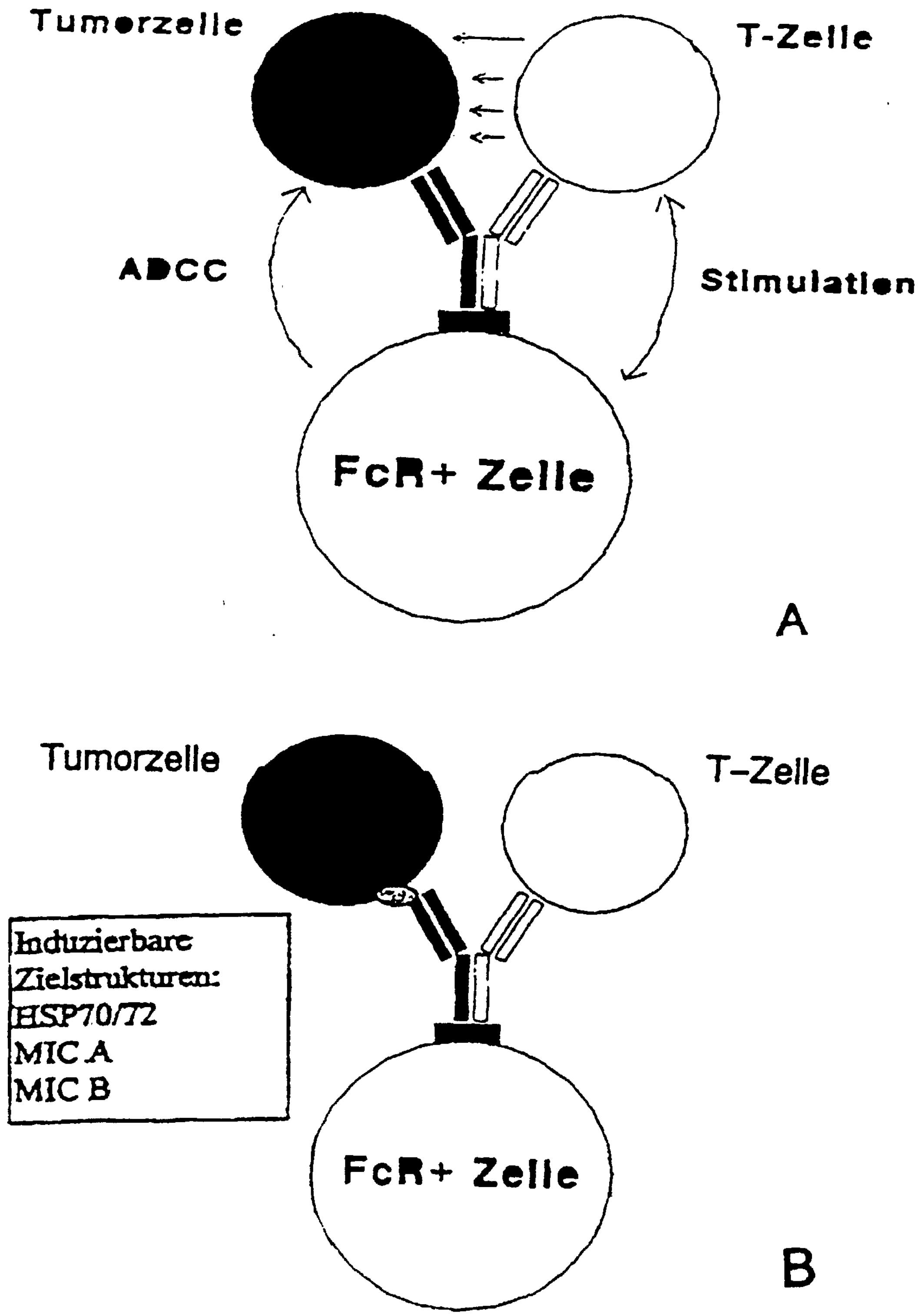
Tumorzelle
T-Zelle
ADCC
Stimulation
FcR+ Zelle
A
Tumorzelle
T–Zelle
Induzierbare
Zielstrukturen:
HSP70/72
MIC A
MIC B
FcR+ Zelle
B